(19) Europäisches Patentamt
European Patent Office
Office européen des brevets

(11) EP 1 070 075 B1

(12) EUROPEAN PATENT SPECIFICATION

(45) Date of publication and mention of the grant of the patent:
**08.10.2003 Bulletin 2003/41**

(21) Application number: **99920633.7**

(22) Date of filing: **09.04.1999**

(51) Int Cl.[7]: **C07F 9/6553**, C07F 15/00, B01J 31/24, C07C 45/50, C07F 9/6558, C07F 9/6568, C07F 9/572, C07B 53/00 // C07M7:00

(86) International application number:
**PCT/EP99/02432**

(87) International publication number:
**WO 99/052915 (21.10.1999 Gazette 1999/42)**

(54) **CHIRAL PHOSPHORATED LIGANDS USEFUL IN CATALYSTS**

CHIRALE PHOSPHORIERTE LIGANDE BRAUCHBAR IN KATALYSATOREN

LIGANDS PHOSPHORES CHIRAUX UTILES DANS DES CATALYSEURS

(84) Designated Contracting States:
**AT BE CH CY DE DK ES FI FR GB GR IE IT LI NL PT SE**

(30) Priority: **10.04.1998 IT MI980773**

(43) Date of publication of application:
**24.01.2001 Bulletin 2001/04**

(73) Proprietor: **CHEMI S.p.A.**
**Cinisello Balsamo (Milano) (IT)**

(72) Inventors:
- **PICCOLO, Oreste**
  **I-23896 Sirtori (IT)**
- **GANCIA, Emanuela**
  **Hitchin, Herts SG5 1PN (GB)**
- **ZALIANI, Andrea**
  **I-20148 Milan (IT)**
- **BONIFACIO, Fausto**
  **I-04100 Latina (IT)**

(74) Representative: **Gervasi, Gemma, Dr. et al**
**NOTARBARTOLO & GERVASI Srl,**
**Corso di Porta Vittoria, 9**
**20122 Milano (IT)**

(56) References cited:
**WO-A-96/01831** **WO-A-97/47633**

- **KRANENBURG M ET AL: "NEW DIPHOSPHINE LIGANDS BASED ON HETEROCYCLIC AROMATICS INDUCING VERY HIGH REGIOSELECTIVITY IN RHODIUM-CATALYZED HYDROFORMYLATION: EFFECT OF THE BITE ANGLE" ORGANOMETALLICS, vol. 14, no. 6, 1 June 1995 (1995-06-01), pages 3081-3089, XP000565317 ISSN: 0276-7333 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 070 075 B1

## Description

The present invention refers to new atropo-isomeric chiral phosphorated ligands having $C_1$ symmetry, the procedure for their preparation, the organometallic complexes containing said phosphorated ligands in optically active form, and the use of these complexes as catalysts in stereoselective organic syntheses.

Stereoselective reactions catalysed by enantiomerically pure complexes of transition metals, such as enantio- and/or diastereo-selective reactions of reduction, isomerization, hydroformylation, hydroboration, hydrosilylation, hydrocyanation, allylation, vinylation, and other reactions of formation of the C-C bond, are the subject of considerable interest from the scientific and application standpoints.

The patent application WO 96/01831 describes chiral diphosphines consisting of a $C_2$-symmetry atropo-isomeric biheterocyclic pentatomic aromatic system, which, by complexation with transition metals, give rise to chiral catalysts capable of inducing good stereoselection in enantio- and/or diastereo-selective reduction and isomerization reactions.

For the use on an industrial scale of chiral organometallic catalysts, in addition to the stereoselectivity induced by these catalysts, of great importance are factors such as their cost, stability, productivity (kg of product per kg of catalyst per day), and the possibility of efficient recycling in the absence of racemization and loss of stereoselection. In addition, there does not exist a catalyst which is suitable for any reaction, nor, given the same reaction, for any substrate.

For example, even though the catalysts containing $C_2$-symmetry atropo-isomeric biheterocyclic ligands described by WO 96/01831 are endowed with a good capacity for inducing stereoselection in the reactions referred to above, they prove less efficient in certain stereoselective reactions, such as hydroformylation, hydrocyanation or hydrosilylation.

Consequently, even though the number of organometallic catalysts is high and constantly increasing, the need is felt for identifying new chiral catalysts that are selective, easy to prepare, economical, stable, provided with high productivity, and may be possibly recycled without racemizing and without losing stereoselectivity.

The search for new and efficient asymmetric catalysts is still based upon the synthesis and experimental verification of a large number of compounds.

Even though an approach of this kind may be fruitful in some cases, it entails numerous disadvantages in terms of work and costs, and often leads to unsatisfactory results.

Now the applicant has unexpectedly found a critical selection of molecular parameters which enables the properties of phosphorated ligands to be foreseen, and hence selective and efficient organometallic catalysts to be synthesized, determining *a priori* the structures of interest, and thus avoiding a purely experimental approach based upon the synthesis and *a posteriori* verification of the properties of the ligands.

A fundamental feature of the present invention hence consists in atropo-isomeric chiral phosphorated ligands of formula (I), having $C_1$ symmetry, in the optically active form or in the racemic form, i.e., as individual atropo-isomers or mixtures of these:

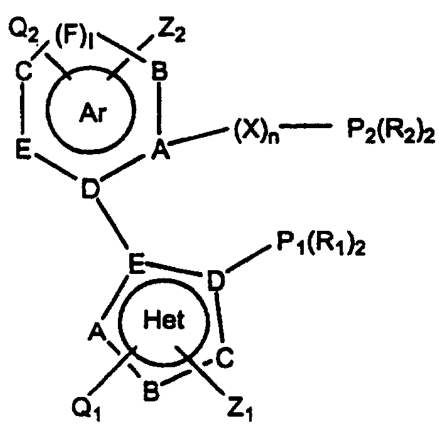

(I)

wherein

the atoms A, B, C, D, E and F, which are equal to or different from one another, are carbon atoms or hetero-atoms chosen from among oxygen, nitrogen and sulphur, which form together an Ar of Het aromatic residue, where Ar is chosen between pentatomic heterocyclic residue and phenyl, and Het is a pentatomic heterocyclic residue, and where

said pentatomic heterocyclic aromatic residue contains 1 or 2 hetero-atoms, equal to or different from one another, selected from the group consisting of -O-, -S- and $-NR_3-$, wherein $R_3$ = H, an alkyl group (for example, $C_1$-$C_6$), an aromatic group (for example, phenyl), a group $-P_1(R_1)_2$, or a nitrogen atom comprised as hetero-atom in the other pentatomic heterocyclic residue belonging to the structure of formula (I) ;
I = 0, 1 ; when I =1, F is a carbon atom ;

$R_1$ and $R_2$, bound to the phosphorous atoms, equal to or different from one another, are selected from a linear, branched or cyclic $C_3$-$C_{10}$ alkyl group, a carbocyclic aromatic group (for example, phenyl or naphthyl), and a heterocyclic aromatic group having 5-6 members in the cycle, containing one or more hetero-atoms (for example, 1-2) chosen among oxygen, sulphur and nitrogen, where said carbocyclic or heterocyclic aromatic group is possibly substituted with one or more groups selected from a linear or branched $C_1$-$C_{10}$ alkyl group, a linear or branched $C_1$-$C_{10}$ alkoxyl group, an halogen, $-COOR_4$, $-SO_3R_4$ and $-NR_5R_6$, where $R_4$ is chosen among H, alkyl (for example, $C_1$-$C_{10}$), aryl (for example, phenyl), alkaline or alkaline-earth metal, $-NH_4^+$ and alkyl ammonium cation having from 4 to 20 carbon atoms ; and where $R_5$ and $R_6$, equal to or different from one another, are H or alkyl (for example, $C_1$-$C_{10}$ alkyl) ; or
**$R_1$ together with the phosphorous atom, or $R_2$ together with the phosphorus atom**, form a heterocycle having 3-6 atoms in the cycle, possibly substituted with linear or branched $C_1$-$C_{10}$ alkyl groups;
X is an -O- group or an $-N(R_7)-$ group, where $R_7$ is chosen among H, alkyl (for example, $C_1$-$C_6$ alkyl) and phenyl;
n may have one of the following values :
is 0 or 1, when Ar is a heterocyclic aromatic residue, and
n is 1, when Ar is phenyl;
$Q_1$, $Q_2$, $Z_1$ and $Z_2$, equal to or different from one another, are selected from the group consisting of H, linear, branched or cyclic $C_1$-$C_{10}$ alkyl, linear or branched $C_1$-$C_{10}$ alkoxyl, a carbocyclic aromatic residue (for example, phenyl) and halogen, or
$Q_1$ taken together with $Z_1$, or $Q_2$ taken together with $Z_2$, form a carbocyclic aromatic ring (for example, phenyl or naphthyl), possibly substituted with one or more T groups (for example, one or two T groups), where T is chosen among halogen, $C_1$-$C_{10}$ alkyl, $C_1$-$C_{10}$ alkokyl, $-COOR_4$, $-SO_3R_4$ and $-NR_5R_6$, where $R_4$ is selected from H, alkyl (for example, $C_1$-$C_{10}$ alkyl), aryl (for example, phenyl), alkaline or alkaline-earth metal, $-NH_4^+$ or alkyl ammonium cation having from 4 to 12 carbon atoms, and where $R_5$ and $R_6$, equal to or different from one another, are selected from H and alkyl (for example, $C_1$-$C_{10}$ alkyl).

The groups $-P_1(R_1)_2$ and $-(X)_n-P_2(R_2)_2$ are bound to the corresponding carbocyclic or heterocyclic aromatic residue by means of a carbon atom of said aromatic residue or by means of a nitrogen atom comprised as hetero-atom in a pentatomic heterocyclic residue.

The ligands in question moreover present:

i) a difference between the residual charges of the phosphorous atoms

$$\Delta Q(P) = Q(P_1) - Q(P_2) > 0.05 \qquad \text{(preferably > 0.15)},$$

where $Q(P_1)$ and $Q(P_2)$ are the values of difference between the number of valence electrons and the number of electrons actually present for the phosphorous atoms $P_1$ and $P_2$;
ii) a cone angle $\beta_n$ ("natural bite angle" according to Casey), ranging from 80° to 130°, preferably from 83° to 120°, defined as preferred chelation angle $P_1$-M-$P_2$, between the phosphorous atoms $P_1$ and $P_2$ and a transition metal M, obtained by minimization of the strain energy of the fragment M(diphosphine), choosing Rh as transition metal;
iii) a value of the barrier of interconversion energy between the two enantiomers of a given ligand

$$\Delta E = E_{trans} - E_{min} \geq 28 \text{ Kcal/mol},$$

where $E_{trans}$ is the energy value for the transition state, and $E_{min}$ is the energy value for the state of minimum energy of the enantiomers, expressed in Kcal/mol.

A further subject of the present invention is the procedure of preparation of the above-mentioned ligands of formula (I), comprising:
one of the following procedure :

A) coupling reaction between aromatic or hetero-aromatic halides with organometallic aryl or hetero-aryl reactants selected from organolithium, organomagnesium, organozinc, and organoboron, in the presence of catalytic quantities of salts or complexes of copper, nickel, or palladium; or
B) cyclisation and aromatisation, with formation of one of the two heterocyclic rings comprised in the structure of

formula (I), of a precursor already containing the other heterocyclic or carbocyclic system ;

in said procedure the introduction of the groups containing the phosphorous atom preceding or following the reaction of formation of the inter-annular bond.

The structure of the compounds of formula (I) in which n is 1, and X is -O- or $-NR_7$ has been identified by setting by approximation that

$$C.3 - P_2 \equiv N- P_2 \equiv O- P_2,$$

i.e., that the phosphorous atom $P_2$ is directly bound to a tetrahedral carbon atom C.3, instead of to oxygen or nitrogen, and hence by using for the bonds $N-P_2$ and $O-P_2$ the same calculation parameters as those used for the bond $C.3 -P_2$.

Once resolved into their optical antipodes, the present atropo-isomeric chiral phosphorated ligands of formula (I), having $C_1$ symmetry, are useful in the preparation of complexes with transition metals, which are in turn useful as catalysts in stereoselective reactions.

Further aspects of the present invention are therefore represented by the organometallic complexes between the optically active form (enanatiomerically pure or at least enriched) of a ligand of formula (I) and a transition metal, the procedure for their preparation, and their use in the preparation of an optically active chiral catalyst. Further subjects of the present invention are the use of the present catalyst in stereoselective (diastereoselective or enantioselective) reactions, and therefore the processes of synthesis for the preparation of organic compounds in the form of stereo-isomers, which comprise at least one strereocontrolled reaction carried out in the presence of one of the present optically active chiral catalysts.

The optically active chiral catalysts of the present invention have unexpectedly been found to be superior to those described by WO 96/01831 in some stereoselective reactions.

Figures 1-3 show the structures of some examples of phosphorated ligands according to the present invention, indicated as compounds (1) - (15).

In the phosphorated ligands of the present invention, the atoms engaged in the bond between the two aromatic cycles are carbon atoms or nitrogen atoms.

The present ligands of formula (I) having $C_1$ symmetry in which Ar is a heterocycle and those in which Ar is phenyl are represented by the following formulas (I)a and (I)b, respectively:

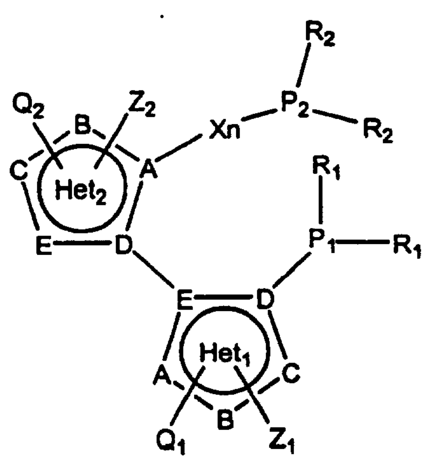

(I)a

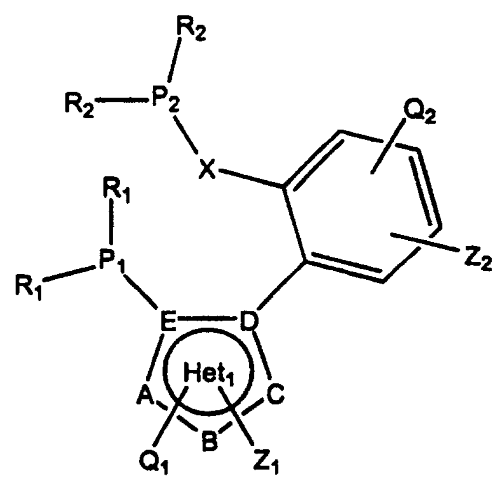

(I)b

wherein

$Het_1$ and $Het_2$ are pentatomic heterocyclic aromatic rings, equal to or different from one another, defined as Het is defined above;

n is 0 or 1; and

X, A, B, C, D, E, $Q_1$, $Q_2$, $Z_1$ and $Z_2$ are as defined above.

The condition that the above-mentioned ligands should have $C_1$ symmetry imposes that the two substituted

aromatic residues present in formula (I) are not mutually specular. Hence, in the case of the ligands of formula (I)a, at least one of the following requirements must be met:

$R_1 \neq R_2$,
$Het_1 \neq Het_2$,
$Q_1 \neq Q_2$,
$Z_1 \neq Z_2$, or
n = 1.

In the case where $Het_1 = Het_2$, $R_1 = R_2$, $Q_1 = Q_2$, $Z_1 = Z_2$, and n = 0, the $C_1$-type asymmetry occurs, for example, when the two pentatomic cyclic residues, even if they derive from the same type of aromatic heterocycle, are bound together via two different relative positions with respect to the hetero-atom, for example via the position 2' of $Het_1$ and the position 3' of $Het_2$.

Examples of Het, $Het_1$ and $Het_2$ heterocyclic residues are thiophene, pyrrole, furan, imidazole, isoxazole, isothiazole, pyrazole and triazole.

When the substituents $Q_1$ and $Z_1$ taken together, or $Q_2$ and $Z_2$ taken together, form a carbocyclic aromatic ring, the Het, $Het_1$ or $Het_2$ pentatomic heterocyclic ring is condensed with phenyl or naphthyl. In this case Het, $Het_1$ or $Het_2$ may be, for example, benzothiophene, naphthothiophene, indole, benzofuran or benzoimidazole.

$Q_1$, $Q_2$, $Z_1$ and $Z_2$ are, for example, methyl.

Examples of heterocyclic aromatic residues present in the ligands of the present invention are 2,5-dimethyl-thien-3-yl, 4,6-dimethyl-benzofur-3-yl, 3-methyl-indol-2-yl, 1-N-methyl-indol-2-yl and benzothien-3-yl.

The carbocyclic aromatic residue is, for example, phenyl.

n is, for example, 0.

When n = 1, X is, for example, -O-.

For example, in certain compounds Ar = phenyl, n = 1 and X = -O-.

The groups $R_1$ and $R_2$ are, for example, phenyl or cyclohexyl, hence $-P_1(R_1)_2$ and $-P_2(R_2)_2$ are, for instance, diphenyl phosphine or dicyclohexyl phosphine.

According to other embodiments of the present invention, the two $R_1$ residues bound together with the atom $P_1$ (or the two $R_2$ residues bound together with the atoms $P_2$) represent a cyclic residue $J_1$ or $J_2$

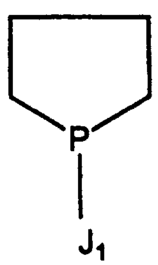

(phospholyl)

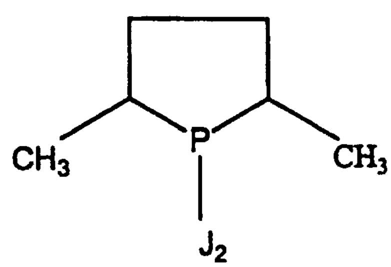

(2',5'-dimethyl-phospholyl)

or a polycyclic aromatic residue, for example, of formula $J_3$

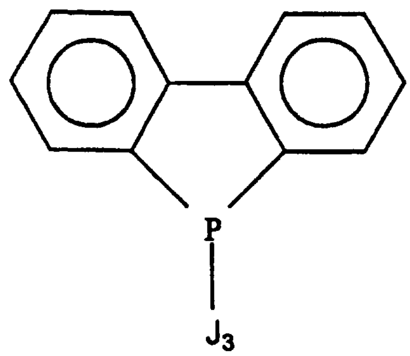

(dibenzophospholyl)

Examples of sub-structures contained in the phosphorated ligands of the present invention are: (4-diphenylphosphine)- or (4-dicyclohexylphosphine)-2,5-dimethylthien-3-yl; (1-N-diphenylphosphine)- or (1-N-dicyclohexylphosphine)-3-methylindol-2-yl; (3-diphenylphosphine)- or (3-dicyclohexylphosphine)-1-N-methylindol-2-yl; 2-(diphenylphosphine)- or 2-(dicyclohexylphosphine)-benzothien-3-yl; 2-(diphenylphosphine-oxy)- or 2-(dicyclohexylphosphine-oxy)-phenyl-1-yl; 4-(diphenylphosphine-oxy)- or 4-(dicyclohexylphosphine-oxy)-2,5-dimethyl-thien-3-yl; 4-(2',5'-dimethylphospholyl)- or 4-(dibenzophospholyl)-2,5-dimethyl-thien-3-yl; 1-N-(2',5'-dimethyl-phospholyl)- or 1-N-(dibenzophospholyl)-3-methyl-indol-2-yl.

Examples of the present $C_1$-symmetry atropo-isomeric ligands are the ligands of formulas (I)c, (I)d, and (I)e represented below:

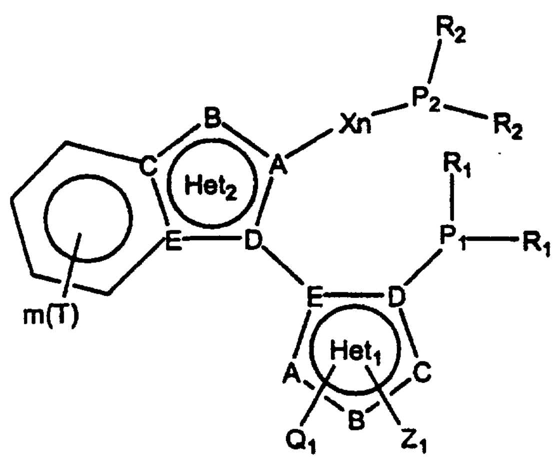

(I)c

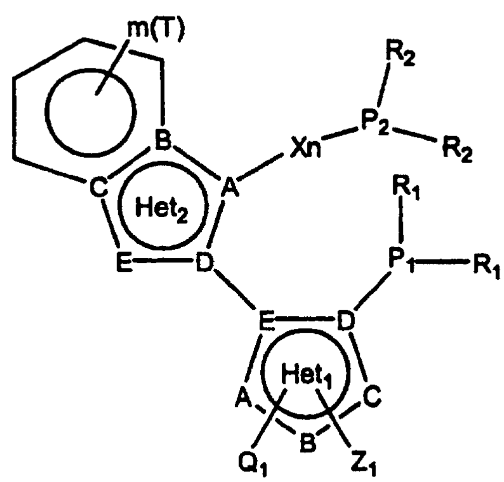

(I)d

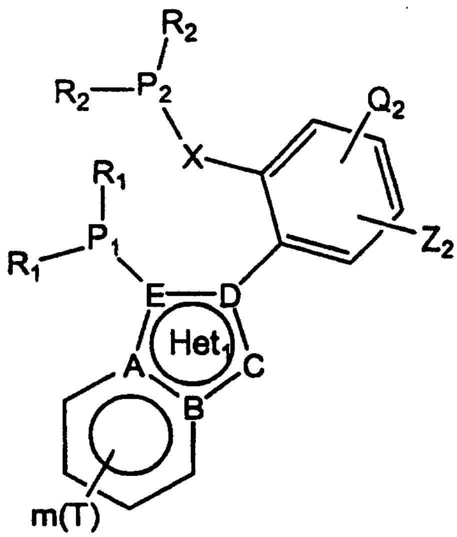

(I)e

In the structures (I)c, (I)d and (I)e, $Het_1$ and $Het_2$ are defined as Het ; A, B, C, D, E, $Q_1$, $Z_1$, $P_1$, $R_1$, $Q_2$, $Z_2$, $P_2$, $R_2$ and T are as defined as for the formula (I) ; m is 0, 1 or 2.

Examples of transition metals contained in the organometallic complexes of the present invention are Rh, Ru, Ir, Pt, Pd and Ni.

Construction of the molecular models, conformational analysis, and calculation of the "natural bite angle" can be carried out by using the S/W program SYBYL, Version 6.2 [Sybyl; Tripos Associates, 193 S. Hasley Road, Suite 363, St. Louis MO 63144].

Minimisation of the structures, calculation of the energy levels associated to the ground state and to the transition state, and the value of the atomic charges can be determined by using the program MOPAC, Version 6.0, Method MNDO [J.P. Stewart, J. Comp. - Aideed Molec. Design, 4 (1), 1990 ; QCPE, Quantum Chemistry Program Exchange - QCMP019 Indiana University - Chemistry Department].

More particularly, the structures of the ligands of formula (I) can be created according to step a) of the present procedure by using the SYBYL modelling software, Version 6.2. Then, according to procedures known to the person skilled in the art, a structural investigation was carried out to determine the minimum-energy conformation associated to each individual structure. The reliability of the forecast of the minimum-energy conformer can then be increased by subjecting the conformations thus identified to a further structural investigation, defined as "optimisation", by using the program MOPAC, Version 6.0, method MNDO, via which the energy levels of the conformers were calculated, as well as the values of the residual charge quantities $Q(P_1)$ and $Q(P_2)$ for the phosphorous atoms $P_1$ and $P_2$, and then the $\Delta Q(P)$ as defined above.

A further parallel optimisation investigation can be carried out, again using the program MOPAC, Version 6.0, method MNDO, to determine the value of the interconversion energy barrier $\Delta E$ between the two enanatiomers (atropo-isomers), or racemisation energy barrier, for each structure of formula (I).

This $\Delta E$, as defined above, corresponds to the maximum possible extension, given by the difference between the energy of the maximum-energy conformers $E_{trans}$ and the energy of the minimum-energy conformer $E_{min}$, for each ligand examined, and can be calculated by imposing that the said maximum energy should be the one associated to the conformer in which the two aromatic rings (the two heterocycles, or the heterocycle and the carbocyclic system) are coplanar.

For the purposes of the present invention, the cone angle $\beta_n$ is as defined in the article by Casey et al., *Isr. J. Chem.*, 30, 299-304, 1990, and is determined uniquely by the steric compression of the ligand structure, and not by the valence angle of the transition metal chosen for the complexation. However, it can be calculated by using a program other than the software program AMBER which was employed according to the said article.

In fact, according to the present procedure, the cone angle can be calculated by using the program SYBYL, Version 6.2, assuming that M = Rh and using the force field parameters of the program TRIPOS, modified by entering the parameters developed for the Rh-diphosphine complexes by M. Kranenburg et al. *[Organometallics,* 14, 3081, 1995]: by means of this modified program, the optimal geometry of the ligand-metal complex can be determined, associating the preferred cone angle to the structure of the minimum-energy conformer.

The parameters developed by M. Kranenburg and entered in the TRIPOS force field, which are used in the procedure of the present invention, are given in the following Tables 1-6, in which:

H = hydrogen; Å = angstrom

P.p = phosphorous atom
C.3 = saturated carbon atom ($sp^3$) bound to the phosphorous
C.ar = aromatic carbon atom bound to the phosphorous
Rh = rhodium; s = single bond; ar =aromatic bond 11

Table 1

| BONDLENGTHS | | | |
|---|---|---|---|
| Atom i | Atom j | Type of bond | Bond length (Å) |
| H | P.p | s | 1.43 |
| C.3 | P.p | s | 1.85 |
| C.ar | P.p | s | 1.83 |
| Rh | P.p | s | 2.315 |

Table 2

| BOND TYPES | | | |
|---|---|---|---|
| Atom i | Atom j | Type of bond | Ambiguity |
| H | P.p | s | no |
| C.3 | P.p | s | no |
| C.ar | P.p | s | no |
| Rh | P.p | s | no |

Table 3

| BENDING ANGLE | | | | |
|---|---|---|---|---|
| Atom i | Atom j | Atom k | Theta | k (Kcal/mol·degrees$^2$) |
| H | P.p | H | 93.4 | 0.02 |
| C.3 | P.p | H | 95 | 0.02 |
| C.ar | P.p | C.3 | 96 | 0.02 |
| Rh | P.p | C.ar | 100 | 0.02 |
| P.p | Rh | P.p | 120 | 0.02 |
| C.ar | P.p | Rh | 109.5 | 0.02 |
| Theta = bending angle between the atoms considered, expressed in degrees<br>k (kcal/mol·degrees$^2$) = bending force | | | | |

Table 4

| STRETCHING ANGLE - Calculation parameters | | | | |
|---|---|---|---|---|
| Atom i | Atom j | Type of bond | L (Å) | k i,j (Kcal/mol) |
| C.3 | P.p | s | 1.85 | 350 |
| H | P.p | s | 1.43 | 700 |
| C.ar | P.p | s | 1.83 | 1000 |
| P.p | Rh | s | 2.315 | 700 |
| L (Å) = bond length in angstrom<br>k i,j = stretching force | | | | |

Table 5

| ROTATIONAL BARRIER - Calculation parameters | | | | | | |
|---|---|---|---|---|---|---|
| Atom i | Atom j | Atom k | Atom l | Type of bond | k (Kcal/mol) | P |
| * | C.3 | P.p | * | s | 0.4 | 3 |
| * | C.ar | P.p | * | s | 1 | 3 |
| * | C.ar | P.p | * | ar | 1 | 3 |
| C.3 | P.p | Rh | P.p | s | 0.2 | 3 |
| C.ar | P.p | Rh | P.p | s | 0.2 | 3 |
| C.ar | C.ar | Rh | P.p | s | 0.2 | 3 |
| C.ar | C.3 | P.p | Rh | s | 0.2 | 3 |
| k = rotational force<br>P = periodicity | | | | | | |

Table 6

| Van der Waals radius | | |
|---|---|---|
| Atom | r (Å) | k (kcal/mol) |
| P.p | 1.8 | 0.314 |
| Rh | 1.844 | 0.63 |
| r (Å) = Van der Waals radius expressed in angstrom<br>k = Van der Waals force | | |

The chemical synthesis of the phosphorated ligands according to the present invention is carried out according to one of the following general procedures, in themselves known:

A) coupling reaction between aromatic or hetero-aromatic halides with organometallic aryl or hetero-aryl reactants, such as organolithium, organomagnesium, organozinc, organoboron, etc., in the presence of catalytic quantities of salts or complexes of copper, nickel, or palladium [see, for example, Takao Sakamoto, Yoshinori Kondo, Nobuo Takazawa, Hiroshi Yamanaka, *J. Chem. Soc.*, Perkin Trans., 1, 1996, Pages 1927-1929];
B) cyclisation and aromatisation, with formation of one of the two heterocyclic rings comprised in the structure of formula (I), of a suitable precursor already containing the other heterocyclic or carbocyclic system.

The introduction of the groups containing the phosphorous atom may precede or follow the reaction of formation of the inter-annular bond.

In the case of phosphine derivatives, for example, one of the following reactions in themselves known will be used:

$$Ar\text{-}[M] + XP(R_1)_2 \rightarrow Ar\text{-}P(R_1)_2$$

$$Ar\text{-}[M] + XP(=O)\ (R_1)_2 \rightarrow Ar\text{-}P(=O)(R_1)_2 \rightarrow Ar\text{-}P(R_1)_2$$

$$Ar\text{-}[M] + (R_2O)_2P(=O)(R_1) \rightarrow Ar_2\text{-}P(=O)(R_1) \rightarrow Ar_2\text{-}PR_1$$

$$Ar\text{-}X + ZP(R_1)_2 \rightarrow Ar\text{-}P(R_1)_2$$

wherein Ar is an aromatic residue comprised in the structure of formula (I) ; [M] is an organometallic group, such as for example Li, MgX, ZnX and an organoboron residue, where X is a halogen ; Z is an alkaline metal, such as Li, Na

and K ; $R_1$ and $R_2$ are alkyl or aryl residues.

In the case of phosphite or aminophosphine derivatives, for example, one of the following reactions, in themselves known, is used :

$$Ar\text{-}OH + XP(R_1)_2 \rightarrow Ar\text{-}OP(R_1)_2$$

$$Ind\text{-}NZ + XP(R_1)_2 \rightarrow Ind\text{-}NP(R_1)_2$$

$$Ind\text{-}NZ + XP(=O)(R_1)_2 \rightarrow Ind\text{-}NP(=O)(R_1)_2 \rightarrow Ind\text{-}NP(R_1)_2$$

$$Ar\text{-}NHR_2 + XP(R_1)_2 \rightarrow Ar\text{-}NR_2P(R_1)_2$$

$$Ar\text{-}X + ZOP(R_1)_2 \rightarrow Ar\text{-}OP(R_1)_2$$

wherein Ar is a carbocyclic aromatic or hetero-aromatic residue comprised in the structure of formula (I) ; Ind is an indole residue ; X is a halogen ; Z is an alkaline metal, such as Li, Na and K, or Z is a MgX group ; $R_1$ is an alkyl or aryl group ; $R_2$ is H or an alkyl or aryl group.

The resolution of the present phosphorated ligands into their optical antipodes is carried out according to techniques in themselves known; for example, by separation on chromatographic column or through membrane, by using a chiral stationary substrate or a chiral eluent, or by means of fractioned crystallisation of a corresponding diastereomeric adduct.

If the present phosphorated ligands comprise basic or acidic groups, for example, amine, carboxyl or sulphone groups, the diastereo-isomeric adducts are, for example, the corresponding salts with enantiomerically pure chiral acids or bases.

Alternatively, the diastereo-isomeric adducts may be, for example, the diastereo-isomeric salts among enantiomerically pure chiral acids, and the phosphinoxides corresponding to the phosphorated ligands, obtained by phosphorous oxidation according to conventional methods : in this case, optical resolution is followed by reduction of the optically active phosphinoxides to phosphine, by means of a treatment with suitable reducing agents, such as sylans, in non-racemising reaction conditions, for example, according to the procedure described in WO 96/01831.

The preparation of the complexes with transition metals of the present phosphorated ligands is carried out according to techniques in themselves known.

The complexes between ligands of formula (I) in the optically active form and transition metals are useful as catalysts in enantio- and/or diastereoselective reactions of reduction, hydroformylation, hydroboration, hydrosilylation, hydrocyanation, allylation, vynylation and other reactions of formation of the C-C bond.

The parameters $\Delta Q$ (P), $\beta_n$ and $\Delta E$ of the present ligands can be measured according to methods known in the art and described in literature.

The $\Delta Q$ (P) data can be obtained from the difference between the electrochemical oxidative potentials (E°) of the two phosphorous atoms, measured by voltammetry (see, for example, T. Benincori, E. Brenna, F. Sanniccolò et al., *J. Organomet. Chem.* **1997,** 529, 445).

The parameter $\beta_n$, that represents the angle between the phosphorous atoms $P_1$ and $P_2$ of the present ligand and a metal M, can be obtained by means of X-ray crystallography (see, for example, M. J. Burk, J. E. Feaster et al., *J. Am. Chem. Soc.* **1993,** 115, 10125; X. Zahng, K. Mashima et al., *J. Chem. Soc.,* Perkin Trans. I **1994**, 2309).

The $\Delta E$ value can be calculated through racemization tests at different temperatures; the optical rotation decrease of enantiomerically pure ligands is measured as a function of time, thus determining the rate constants K at different temperatures; the Arrhenius plot of In K versus 1/T, wherein T is the temperature, leads to the activation data $\Delta E$.

There follow a number of examples given to provide a non-limiting illustration of the present invention.

EXPERIMENTAL PART

**Calculation of the parameters of some phosphorated ligands**

The phosphorated ligands having the structures from (1) to (15) illustrated in Figures 1-3, a calculated interconversion energy barrier = 28 Kcal/mole, and the calculated values of ΔQ(P) and of the natural bite angle according to Casey as given in the following Table 7, have been identified :

Table 7

| Compound | ΔQ(P) | Natural bite angle |
|---|---|---|
| (1) | 0.07 | 86.7 |
| (2) | 0.35 | 97.3 |
| (3) | 0.47 | 108.6 |
| (4) | 0.18 | 97.1 |
| (5) | 0.37 | 88.3 |
| (6) | 0.23 | 83.7 |
| (7) | 0.46 | 97.5 |
| (8) | 0.23 | 93.3 |
| (9) | 0.41 | 99.7 |
| (10) | 0.18 | 87.3 |
| (11) | 0.27 | 98.7 |
| (12) | 0.24 | 84.4 |
| (13) | 0.20 | 118.8 |
| (14) | 0.45 | 104.1 |
| (15) | 0.27 | 99.3 |

**Preparation of intermediate compounds**

EXAMPLE 1

Preparation of 3-diphenyl phosphine-2,5-dimethyl-thiophene

Into a flask, in the following order are introduced : 13.6 ml of water, 4.1 g of sodium iodate, 7.3 g of iodine, 26 ml of acetic acid, 91 ml of ethyl acetate, and 7.8 g of 2,5-dimethyl-thiophene. The mixture is kept under stirring at 25°C, while 3.2 g of 96% sulphuric acid are fed in slowly drop by drop The mixture is then kept under stirring for 10 hours, cooled down to 15°C, and an aqueous solution of sodium chloride (10 g in 68.5 ml) is added. The aqueous phase is separated, and the organic phase is washed, in order, with an aqueous solution of sodium chloride (10 g in 68.5 ml), an alkaline solution of sodium hyposulphite (6.8 g in 70 ml of 1% sodium hydroxide), and again with an aqueous solution of sodium chloride (10 g in 68.5 ml). The organic phase is then dried on sodium sulphate and concentrated to yield 16.4 g of crude 3-iodo-2,5-dimethylthiophene. This residue, in inert atmosphere, is treated with 50 ml of DMF, and the following are added: 8.8 g of potassium acetate, 2 mg of palladium acetate, and 13.8 ml of diphenyl phosphine. The mixture is heated up to approximately 130°C and kept at this temperature until the reaction is completed (approximately 15 hours). The mixture is then cooled to approximately 30°C and diluted with 20 ml of water and 300 ml of methylene chloride. The dichloromethylene phase is separated and washed with 30 ml of water. After concentration to dry residue, 18.5 g are obtained of 3-diphenyl phosphine-2,5-dimethyl-thiophene.

EXAMPLE 2

Preparation of 3-dicyclohexyl phosphine-2.5-dimethyl-thiophene

100 ml of a t-BuLi solution 1.5 M in penthane are fed drop by drop, in inert atmosphere and under stirring, into a solution containing 33.1 g of 3-iodo-2,5-dimethylthiophene prepared according to Example 1 and 18.7 g of tetramethylendiamine in 150 ml of THF anhydrous, at -50°C. The temperature of the mixture is made to rise to -20°C in 30 minutes. A solution of chlorodicyclohexyl-phosphine (36 g) in 40 ml of THF is then fed in drop by drop, and the mixture is kept under stirring while the temperature is brought to 20°C in 4 hours. The mixture is then treated with 50 ml of water and concentrated under vacuum. The residue is treated with 300 ml of methylene chloride. The dichloromethane phase is washed with water (30 ml x 2), then concentrated to residue to yield 28.5 g of crude 3-dicyclohexyl phosphine-2,5-dimethylthiophene. The product is purified by means of silica gel chromatography.

EXAMPLE 3

Prepartion of 3-diphenylphosphinyl-4-bromo-2,5-dimethyl-thiophene

Into a flask, in the following order are inserted : 1.5 g of 3-diphenyl phosphine-2,5-dimethyl-thiophene prepared according to Example 1 and 32 ml of methylene chloride. The mixture is kept stirred at -10°C, and at the same time 2.5 g of N-BROMOSUCCINIMIDE are added slowly in portions. The mixture is then kept under stirring for 15 h, at 25°C, then refluxed after addition of a further 1.3 g of N-BROMOSUCCINIMIDE. After a further 20 h of reaction, 20 ml of water are added, and the phases are separated. The organic phase, re-united to the dichloromethane extract (15 ml) of the aqueous phase, is washed with an aqueous solution of sodium chloride (2 g in 15 ml). The organic phase is then dried on sodium sulphate and concentrated. The residue obtained is purified using silica chromatography to yield 0.9 g of 3-diphenylphosphinyl-4-bromo-2,5-dimethylthiophene.

EXAMPLE 4

Preparation of 3-dicyclohexylphosphinyl-4-bromo-2,5-dimethyl-thiophene

Proceeding as in Example 3 and using 1.8 g of 3-dicyclohexyl phosphine-2,5-dimethyl-thiophene instead of 1.5 g of 3-diphenyl phosphine-2,5-dimethylthiophene, 1.2 g of 3-dicyclohexylphosphinyl-4-bromo-2,5-dimethyl-thiophene are obtained.

**Preparation of phosphorated ligands of the invention**

EXAMPLE 5

Preparation of (+) and (-) 4-diphenyl phosphine-3-[3'(4'-dicyclohexyl phosphine-2',5'-dimethyl(thienyl)]-2,5-dimethyl-thiophene [compound (15)]

A solution of 3-dicyclohexylphosphinyl-4-bromo-2,5-dimethyl-thiophene (3.4 g) prepared according to Example 4 in 20 ml of diethyl ether is fed drop by drop in inert atmosphere into 5 ml of a t-BuLi solution 1.5 M in penthane, at -30°C. The mixture is kept under stirring for 2 h, then 2.5 g of zinc iodide are added to it, and the mixture is allowed to warm up to room temperature. Then a solution of 3-diphenylphosphinyl-4-bromo-2,5-dimethyl-thiophene (3.4 g), prepared according to Example 3, and palladium tetrakistriphenyl phosphine (87 mg) in 20 ml of anhydrous tetrahydrofuran is added to it, and the mixture is refluxed until completion of the reaction. The mixture is then treated with 200 ml of water, vacuum-concentrated to a small volume, and the residue treated with 200 ml of toluene; the organic phase is separated and washed with 30 ml of water, filtered on celite and concentrated to yield 2.8 g of crude (±) 4-diphenylphosphinyl-3-[3'-(4'-dicyclohexylphosphinyl-2',5'-dimethyl)thienyl]-2.5-dimethyl-thiophene. The product is purified via silica chromatography, and resolved in its optical antipodes by crystallization of the diastereo-isomeric salts, using enantiomerically pure dibenzoyltartaric acid, for example, according to the procedure described in WO 96/01831. The diastereo-isomerically pure adducts are then unblocked using sodium hydroxide and reduced with trichlorosilane, according to the procedure described in Example 2 of the patent application WO 96/01831, thus yielding approximately 0.7 g of (+) - and (-)-4-diphenyl phosphine-3-[3'(4'-dicyclohexyl phosphine-2',5'-dimethyl)thienyl]-2,5-dimethyl-thiophene.

Alternatively, starting from the racemic diphosphine oxide, the racemic disphosphine is obtained by reduction with trichlorosilane, and is resolved via HPLC on stationary chiral phase.

EXAMPLE 6

Preparation of (+) and (-) 2-diphenyl phosphine-3-[3'(4'-dicyclohexyl phosphine-2',5'-dimethyl(thienyl)]-4,6-dimethyl-benzofuran [compound (2)]

A solution of 3-dicyclohexylphosphinyl-4-bromo-2,5-dimethyl-thiophene (3.4 g) prepared according to Example 4 in 20 ml of diethyl ether is fed drop by drop in inert atmosphere into 5 ml of a 1.6 M of t-BuLi solution in penthane at -30°C; the mixture is kept under stirring for 2 h, then 2.5 g of zinc iodide are added to it, and the mixture is allowed to warm up to room temperature. Then a solution of 3-bromo-4,6-dimethyl-benzofuran (1.7 g), prepared according to

Example 23 of the patent application WO 96/01831, and palladium tetrakis-triphenylphosphine (57 mg) in 20 ml of anhydrous tetrahydrofuran is added, and the mixture is refluxed until the reaction is completed. The mixture is then filtered on celite and concentrated under vacuum ; the residue is treated with 30 ml of diethyl ether, and the solution, in inert atmosphere, is fed drop by drop into 5 ml of a t-BuLi solution 1.6 M in penthane at the temperature of -30°C ; then 1.4 ml of chlorodiphenyl phosphine is added, and the reaction mixture is allowed to reconstitute at room temperature. After hydrolysis with water, the organic phase is separated and concentrated under reduced pressure; the residue is treated with xylene and reduced with trichlorosilane according to the procedure mentioned previously, to yield 2.5 g of (±) 2-diphenyl phosphine-3-[3'-(4'-dicyclohexylphosphinyl-2',5'-dimethyl)thienyl]-4,6-dimethyl-benzofuran, which is resolved via HPLC on chiral stationary phase.

EXAMPLE 7

Preparation of (+) and (-) 2-diphenyl phosphine-3-[3'(4'-diphenyl phosphine-2',5'-dimethyl(thienyl)]-4,6-dimethyl-benzofuran [compound (1)]

The procedure of Example 6 is repeated using 3.3 g of 3-diphenylphosphinyl-4-bromo-2,5-dimethyl-thiophene, prepared as described in Example 3, instead of the 3.4 g of 3-dicyclohexylphosphinyl-4-bromo-2,5-dimethyl-thiophene, to recover 2.2 g of racemic 2-diphenyl phosphine-3-[3'(4'-diphenyl phosphine-2',5'-dimethyl(thienyl)]-4,6-dimethyl-benzofuran, which is resolved into its optical antipodes by means of HPLC on chiral stationary phase.

EXAMPLE 8

Preparation of (+) and (-) N-diphenyl phosphine-2-[3'(4'-diphenyl phosphine-2',5'-dimethyl(thienyl)]-3-methyl-indole [compound (6)]

To a solution of 4-bromo-2,5-dimethyl-3-propionyl-thiophene (12 g) and phenylhydrazine (34.9) in 250 ml of ethanol are added 61 ml of acetic acid. The mixture is reflux-heated for 4 h, then concentrated under reduced pressure and the residue treated with methylene chloride; the organic phase is washed with a saturated solution of sodium bicarbonate and subsequently with water until neutral pH is obtained. The organic phase is concentrated under vacuum, and the crude reaction product is purified by means of silica chromatography to yield the 4-bromo-2,5-dimethyl-3-propionyl-thiophene phenylhydrazone, which is dissolved in 350 ml of isopropanol/HCl (7.5 M) and kept stirred at room temperature until the reaction is completed. The solvent is removed under reduced pressure, and the residue is treated with methylene chloride. The organic phase is subjected to washings with a saturated solution of sodium bicarbonate, then with water, and finally concentrated under reduced pressure to yield 4.7 g of 2-[3'(4'-bromo-2',5'-dimethyl)-thienyl]-3-methyl-indole.

Into a solution of 4 g of indole derivative thus prepared in 150 ml of anhydrous diethyl ether and 2.2 ml of N,N,N',N'-tetramethylethylenediamine, cooled to -60°C, 16 ml of a t-BuLi solution 1.5 M in penthane are carefully fed in drop by drop. The mixture is allowed to reconstitute at -30°C, and 4.9 ml of chlorodiphenyl phosphine are added to it. After being kept under stirring overnight at room temperature, the mixture is treated with water and concentrated to a small volume; the residue is treated with 150 ml of methylene chloride, and the organic phase washed with water. The solvent is removed to yield 3.3 g of racemic N-diphenyl phosphine-2-[ 3'(4'-diphenyl phosphine-2',5'-dimethyl)-thienyl]-3-methyl-indole, which is resolved into its optical antipodes using HPLC on chiral stationary phase.

EXAMPLE 9

Preparation of the complex obtained from $[Rh(1,5\text{-}COD)_2]ClO_4$ and compound (+)(15)

In argon atmosphere, equimolar solutions of $[Rh(1,5\text{-}COD)_2]ClO_4$ (COD = cyclooctadiene) and of the optically pure ligand (+)(15) in dichloromethane are prepared; these two solutions are then mixed and kept under stirring for 30 minutes. The solution is then concentrated under reduced pressure to yield the Rh complex containing the chiral diphosphine which is used as such without further purification in the enantioselective reduction of olefins. It is assumed that the complex obtained has the following structure:

$[Rh(1,5\text{-}COD)(compound\ (+)(15))]ClO_4$.

Using the same procedure, similar complexes of rhodium were prepared with the other optically active phosphines of Table 7.

EXAMPLE 10

Preparation of the complex obtained from $[Ir(1,5\text{-}COD)Cl]_2$, compound (+)(15), and tetrabutylammonium iodide

In argon atmosphere, a solution is prepared of toluene/methanol 1/1 (3 ml) containing 2.5 x $10^{-3}$ mmol of $[Ir(1,5\text{-}COD)Cl]_2$ and 6.0 x $10^{-3}$ mmol of optically pure ligand (+)(15). After 30 minutes, 1 x $10^{-2}$ mmol of tetrabutylammonium iodide are added under stirring. The solution thus obtained is used as such without further purification in the enantioselective reduction of imines. It is assumed that the complex obtained has the following structure: [Ir(1,5-COD)(compound (+)(15))]).

Using the same procedure, similar complexes of iridium were prepared with the other optically active phosphines of Table 7.

EXAMPLE 11

Preparation of the complex obtained from $[Ir(1,5\text{-}COD)Cl]_2$ and compound (+)(15).

In argon atmosphere, a solution is prepared of diethyl ether (3 ml) containing 2.5x$10^{-3}$ mmol of $[Ir(1,5\text{-}COD)Cl]_2$ and 5.0x$10^{-3}$ mmol of optically pure ligand (+)(15). After 1 h under stirring, the solution thus obtained is used as such without further purification in the enanatioselective hydrosilylation of chetones. It is assumed that the complex obtained has the following structure:
[Ir(1,5-COD)(compound (+)(15))]Cl.

Using the same procedure, similar complexes of iridium were prepared with the other optically active phosphines of Table 7.

EXAMPLE 12

Preparation of the complex obtained from $[Ru(p\text{-}cymene)I_2]_2$ and compound (+)(15).

In argon atmosphere, a solution is prepared of methylene chloride/methanol 8/3 (11 ml) containing 1.6x$10^{-2}$ mmol of $[Ru(p\text{-}cymene)I_2]_2$ and 3.5x$10^{-2}$ mmol of optically pure ligand (+)(15); after 60 minutes at reflux under stirring, the mixture thus obtained is concentrated under reduced pressure to yield a residue containing the complex, which is used as such without further purification, dissolved in methanol or ethanol, in the enantioselective reduction of carbonyl compounds. It is assumed that the complex obtained has the following structure:
[Ru(p-cymene)(compound (+)(15) I)]I.

Using the same procedure, similar complexes of ruthenium were prepared with the other optically active phosphines of Table 7.

EXAMPLE 13

Preparation of the complex obtained from $[Rh(acac)(CO)_2]$ and compound (+)(2)

Into an autoclave in argon atmosphere are introduced a toluene solution (10 ml) containing 2.0 x $10^{-2}$ mmol of $[Rh(acac)(CO)_2]$ and 2.2x$10^{-2}$ mmol of optically pure ligand (+)(2). The autoclave is purged, loaded with $CO/H_2$ 1/1 (pressure, approximately 20 bar) and kept at room temperature for 15 h to form the active catalyst suitable for enantioselective hydroformylation reactions. It is assumed that the complex obtained has the following structure: [H Rh (compound (+)(2)$(CO)_2$].

Using the same procedure, similar complexes of rhodium were prepared with the other optically active phosphines of Table 7.

EXAMPLE 14

Preparation of the complex obtained from $NiCl_2$ and compound (+)(15)

A dichloromethane solution (10 ml) containing 4.2 mmol of optically pure ligand (+)(15) is added under stirring to a 4.2 mmol solution of hexahydrated $NiCl_2$ in 30 ml of ethanol. After 1 h the mixture is concentrated to a small volume, and the residue is squashed with ethanol and subsequently dried under vacuum.

The complex is used as such in enantoselective reactions of formation of C-C bonds. It is assumed that the complex obtained has the following structure:

$[NiCl_2$(compound (+)(15)].

Using the same procedure, similar complexes of nickel were prepared with the other optically active phosphines of Table 7.

EXAMPLE 15

Preparation of the complex obtained from $PdCl_2$(benzonitrile)$_2$ and compound (+)(15)

A dichloromethane solution (10 ml) containing 2.6 mmol of optically pure ligand (+)(15) and 2.6 mmol of $PdCl_2$ (benzonitrile)$_2$ is kept under stirring for 1 h at room temperature. The mixture is concentrated to a small volume and the residue is squashed with ethanol and subsequently dried under vacuum.

The complex obtained is used as such in enantioselective reactions of formation of C-C bonds. It is assumed that the complex obtained has the following structure:

$[PdCl_2$(compound (+)(15)].

Using the same procedure, similar complexes of palladium were prepared with the other optically active phosphines of Table 7.

## Claims

1. An atropo-isomeric chiral phosphorated ligand of formula (I), having $C_1$ symmetry, in the optically active form or in the racemic form

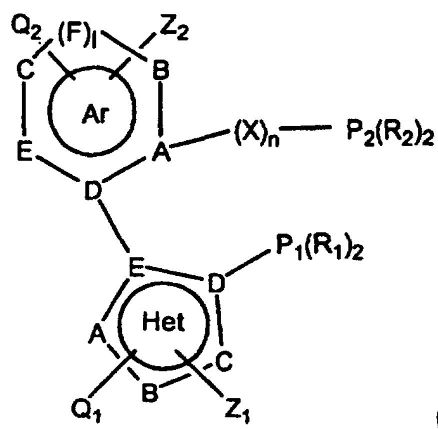

(I)

wherein

the atoms A, B, C, D, E and F, equal to or different from one another, are carbon atoms or hetero-atoms chosen from among oxygen, nitrogen and sulphur, which form together an Ar or Het aromatic residue, where Ar is chosen between pentatomic heterocyclic residue and phenyl, and Het is a pentatomic heterocyclic residue, and where said pentatomic heterocyclic aromatic residue contains 1 or 2 hetero-atoms, equal to or different from one another, selected from the group consisting of -O-, -S- and $-NR_3-$, wherein $R_3$ = H, an alkyl group, an aromatic group, a group $-P_1(R_1)_2$, or a nitrogen atom comprised as hetero-atom in the other pentatomic heterocyclic residue belonging to the structure of formula (I);

I = 0, 1 ; when I =1, F is a carbon atom ;

$R_1$ and $R_2$, bound to the phosphorous atoms, equal to or different from one another, are selected from a linear, branched or cyclic $C_3$-$C_{10}$ alkyl group, a carbocyclic aromatic group, and a heterocyclic aromatic group having 5-6 members in the cycle, containing one or more hetero-atoms chosen among oxygen, sulphur and nitrogen, where said carbocyclic or heterocyclic aromatic group is optionally substituted with one or more groups selected from a linear or branched $C_1$-$C_{10}$ alkyl group, a linear or branched $C_1$-$C_{10}$ alkoxyl group, an halogen, $-COOR_4$, $-SO_3R_4$ and $-NR_5R_6$, where

$R_4$ is chosen among H, alkyl, aryl, alkaline or alkaline-earth metal, $-NH_4^+$ and alkyl ammonium cation having from 4 to 20 carbon atoms; and where $R_5$ and $R_6$, equal to or different from one another, are H or alkyl ; or

$R_1$ and $R_2$ together with the phosphorus atom, form a heterocycle having 3-6 atoms in the cycle, optionally substituted with linear or branched $C_1$-$C_{10}$ alkyl groups;
X is an -O- group or an -N($R_7$)- group, where $R_7$ is chosen among H, alkyl and phenyl;
n is 0 or 1, when Ar is a heterocyclic aromatic residue;
n is 1, when Ar is phenyl ;
$Q_1$, $Q_2$, $Z_1$ and $Z_2$, equal to or different from one another, are selected from the group consisting of H, linear, branched or cyclic $C_1$-$C_{10}$ alkyl, linear or branched $C_1$-$C_{10}$ alkoxyl, phenyl and halogen, or
$Q_1$ taken together with $Z_1$, or $Q_2$ taken together with $Z_2$, form a carbocyclic aromatic ring selected from phenyl and naphthyl, said carbocyclic aromatic ring being optionally substituted with one or more T groups, where T is chosen among halogen, $C_1$-$C_{10}$ alkyl, $C_1$-$C_{10}$ alkokyl, -$COOR_4$, -$SO_3R_4$ and -$NR_5R_6$, where $R_4$ is selected from H, $C_1$-$C_{10}$ alkyl, phenyl, alkaline or alkaline-earth metal, -$NH_4^+$ or $C_4$-$C_{12}$ alkyl ammonium cation, and where $R_5$ and $R_6$, equal to or different from one another, are selected from H and $C_1$-$C_{10}$ alkyl ; and wherein
-$P_1(R_1)_2$ and -$(X)_n$-$P_2(R_2)_2$ are bound to the corresponding carbocyclic or heterocyclic aromatic residue by means of a carbon atom of said aromatic residue or by means of a nitrogen atom comprised as hetero-atom in a pentatomic heterocyclic residue;
said phosphorated ligand further having:

i) a difference between the residual charges of the phosphorous atoms

$$\Delta Q(P) = Q(P_1) - Q(P_2) > 0.05,$$

where $Q(P_1)$ and $Q(P_2)$ are the values of difference between the number of valence electrons and the number of electrons actually present for the phosphorous atoms $P_1$ and $P_2$,
ii) a cone angle $\beta_n$ ("natural bite angle" according to Casey) ranging from 80° to 130°, defined as preferred chelation angle $P_1$-M-$P_2$ between the phosphorous atoms $P_1$ and $P_2$ and a transition metal M,
iii) an energy barrier value of interconversion between the two enantiomers of a given ligand

$$\Delta E = E_{trans} - E_{min} \geq 28 \text{ Kcal/mol},$$

where $E_{trans}$ is the energy value for the transition state, and $E_{min}$ is the value associated to the state of minimum energy of the enantiomers, expressed in Kcal/mol.

**2.** The phosphorated ligand according to claim 1, wherein

i) said difference $\Delta Q(P) = Q(P_1) - Q(P_2)$ is > 0.15 ;
ii) said "natural bite angle" $\beta_n$ ranging from 83° and 120°.

**3.** The phosphorated ligand according to claim 1, wherein said phosphorated ligand is chosen between a ligand of formula (I)a and a ligand of formula (I)b :

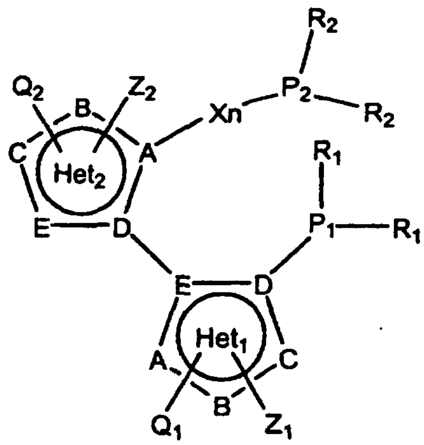

(I)a

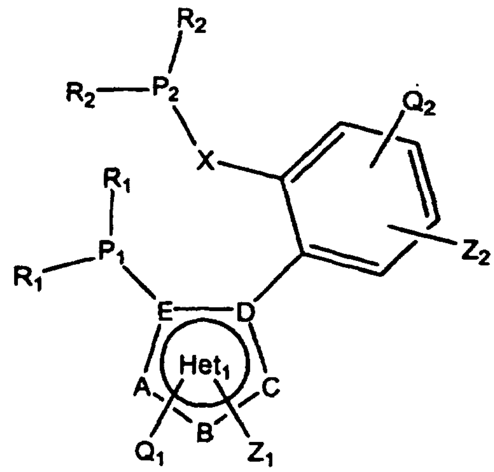

(I)b

where

$Het_1$ and $Het_2$ are pentatomic heterocyclic aromatic rings, equal to or different from one another, defined as Het in claim 1 ;

n is 0 or 1 ;

X, A, B, C, D, E, $Q_1$, $Q_2$, $Z_1$ and $Z_2$ are as defined in claim 1.

**4.** The phosphorated ligand according to claim 1, wherein said heterocyclic residue is selected from the group consisting of thiophene, pyrrole, furan, imidazole, isoxazole, isothiazole, pyrazole and triazole.

**5.** The phosphorated ligand according to claim 1, wherein $Q_1$ taken together with $Z_1$, or $Q_2$ taken together with $Z_2$, form a carbocyclic ring, and Het is condensed with phenyl or naphthyl.

**6.** The phosphorated ligand according to claim 5, wherein said heterocyclic ring Het condensed with phenyl is selected from the group consisting of benzothiophene, naphthothiophene, indole, benzofuran and benzoimidazole.

**7.** The phosphorated ligand according to claim 1, wherein said phosphorated ligand is chosen from a ligand of formula (I)c, (I)d and (I)e :

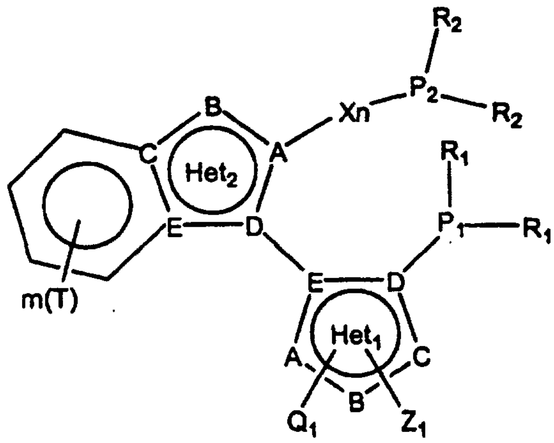

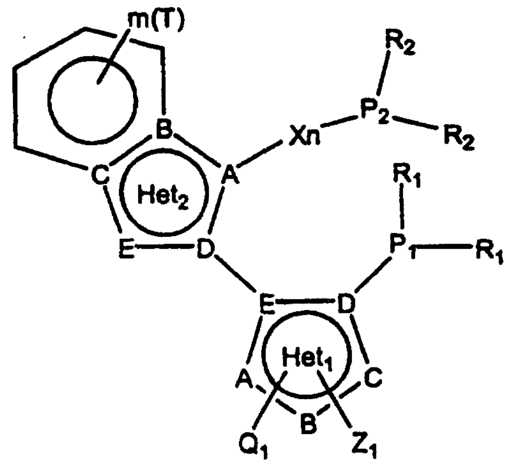

(I)c

(I)d

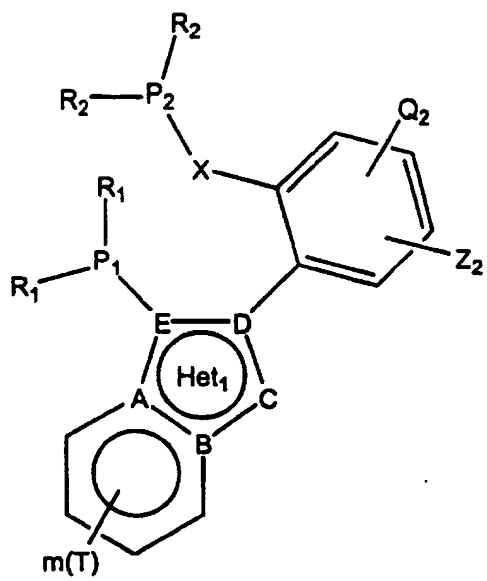

(I)e

wherein $Het_1$ and $Het_2$ are defined as Het in claim 1 ;
A, B, C, D, E, $Q_1$, $Z_1$, $P_1$, $R_1$,$Q_2$, $Z_2$, $P_2$, $R_2$ and T are as defined in claim 1 for formula (I) ;
m is 0, 1 or 2.

**8.** The phosphorated ligand according to claim 1, wherein said heterocyclic aromatic residue is selected from the group consisting of 2,5-dimethyl-thien-3-yl, 4,6-dimethyl-benzofur-3-yl, 3-methyl-indol-2-yl, 1-N-methyl-indol-2-yl, and benzothien-3-yl ; and said carbocyclic aromatic residue is phenyl.

**9.** The phosphorated ligand according to claim 1, wherein said groups $-P_1(R_1)_2$ and $-P_2(R_2)_2$ are selected from diphenyl phosphine, dicyclohexyl phosphine, $J_1$, $J_2$ and $J_3$, where $J_1$, $J_2$ and $J_3$ have the following formulas :

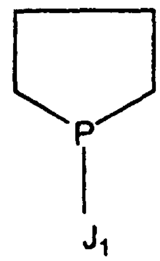

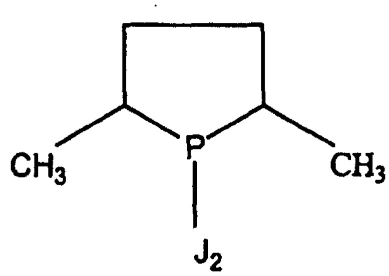

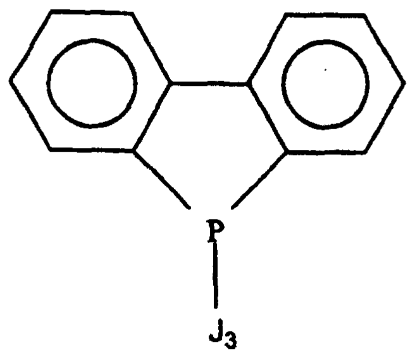

10. The phosphorated ligand according to claim 1, containing one of the following sub-structures : (4-diphenylphosphine)- or (4-dicyclohexylphosphine)-2,5-dimethyl-thien-3-yl; (1-N-diphenylphosphine)- or (1-N-dicyclohexylphosphine)-3-methylindol-2-yl; (3-diphenylphosphine)- or (3-dicyclohexylphosphine)-1-N-methylindol-2-yl; 2-(diphenylphosphine)- or 2-(dicyclohexylphosphine)-benzothien-3-yl; 2-(diphenylphosphine-oxy)- or 2-(dicyclohexylphosphine-oxy)-phenyl-1-yl; 4-(diphenylphosphine-oxy)- or 4-(dicyclohexylphosphine-oxy)-2,5-dimethyl-thien-3-yl ; 4-(2',5'-dimethyl-phospholyl)- or 4-(dibenzophospholyl)-2,5-dimethyl-thien-3-yl ; 1-N-(2',5'-dimethyl-phospholyl)- or 1-N-(dibenzophospholyl)-3-methyl-indol-2-yl.

11. The phosphorated ligand according to claim 1, wherein said phosphorated ligand is chosen from the compounds from (1) to (15).

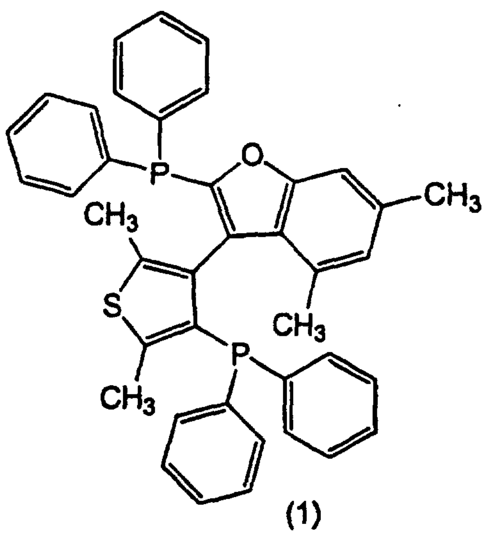

(1)

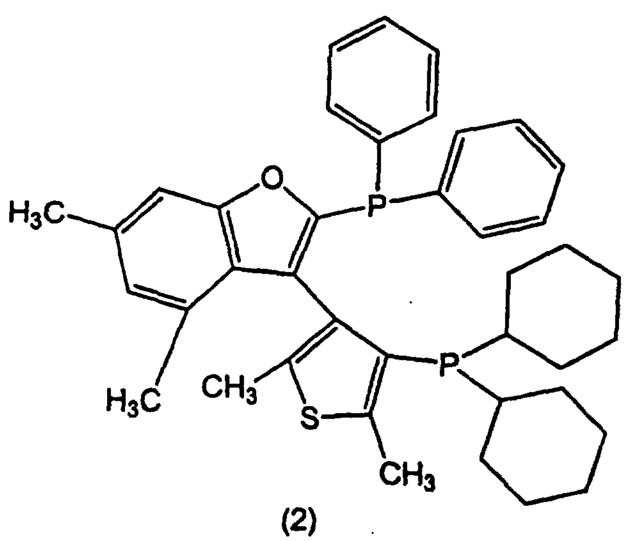

(2)

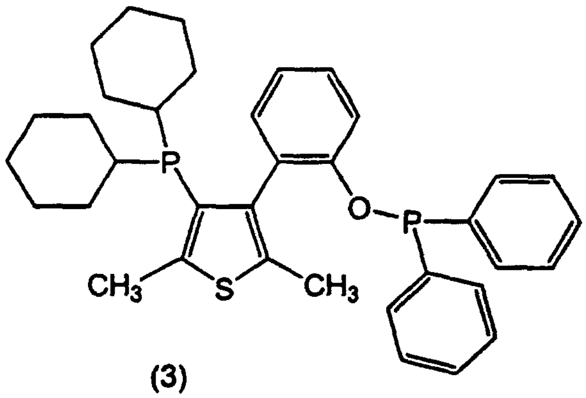

(3)

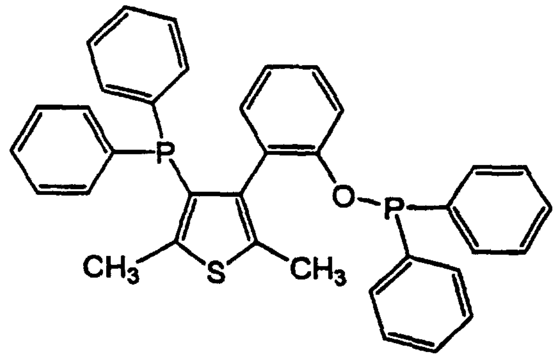

(4)

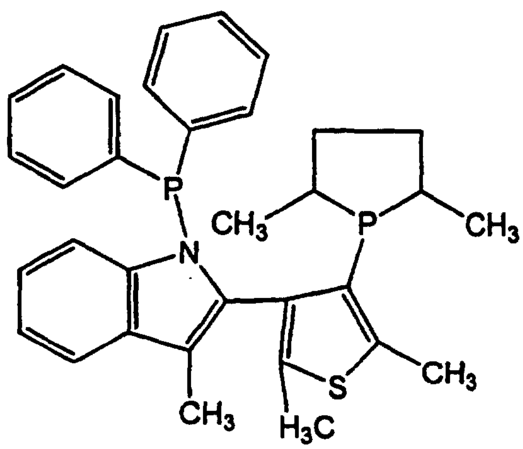

(5)

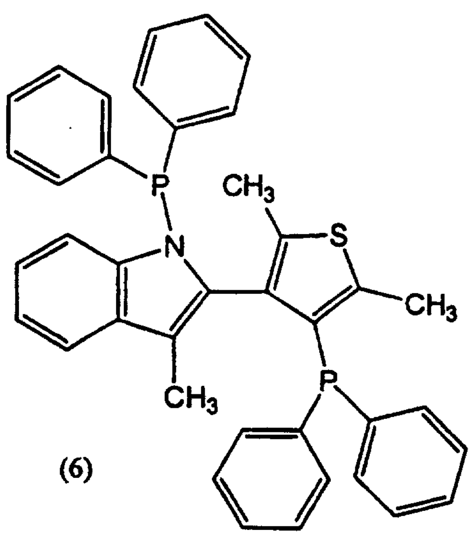

(6)

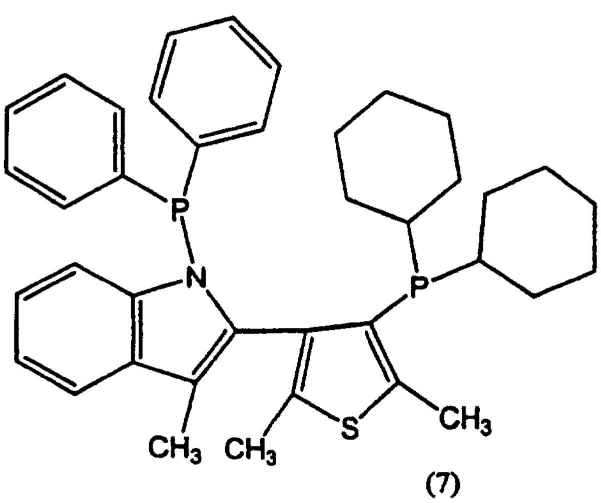

(7)

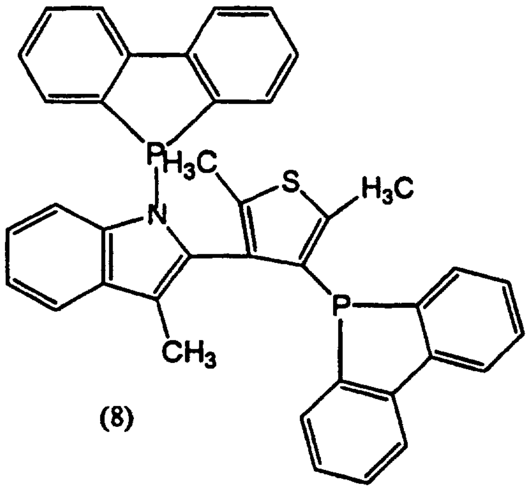

(8)

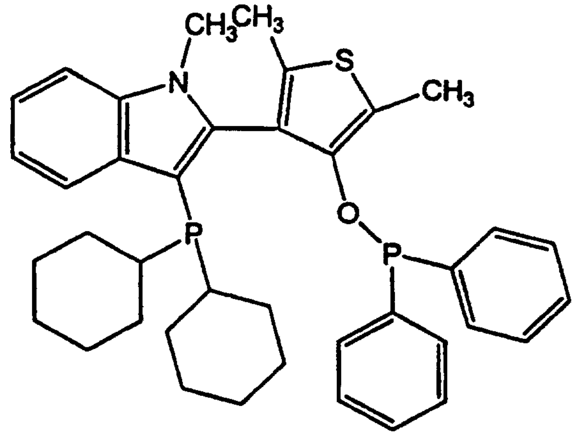

(9)

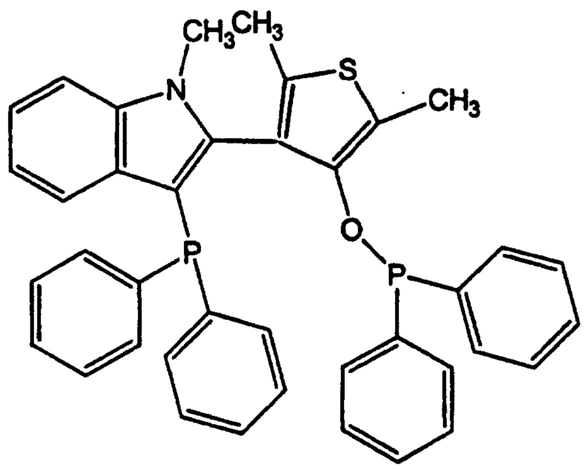

(10)

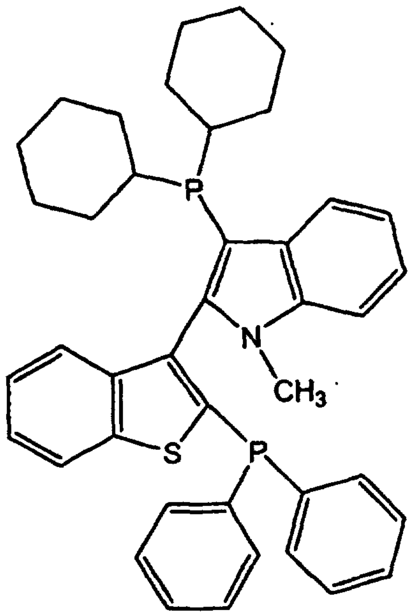

(11)

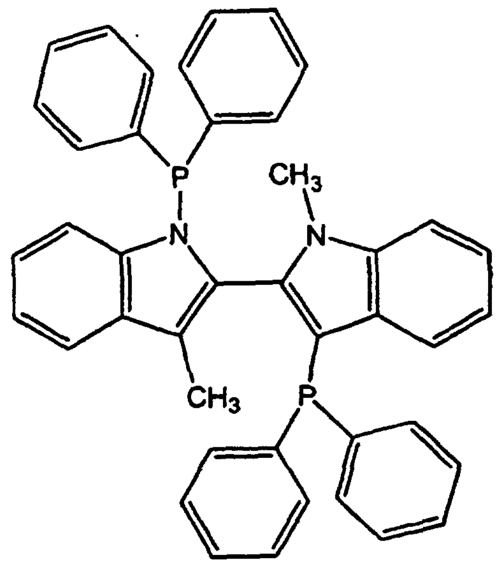

(12)

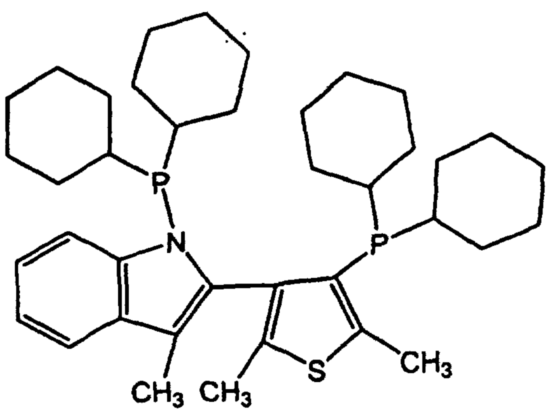

(13)

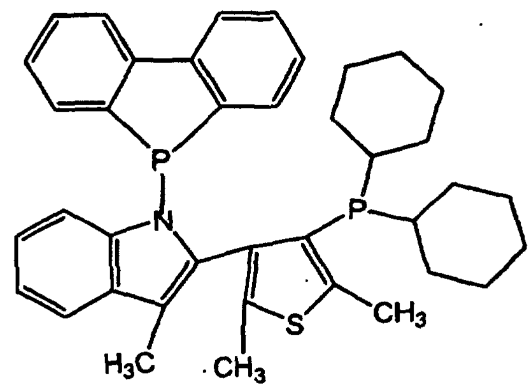

(14)

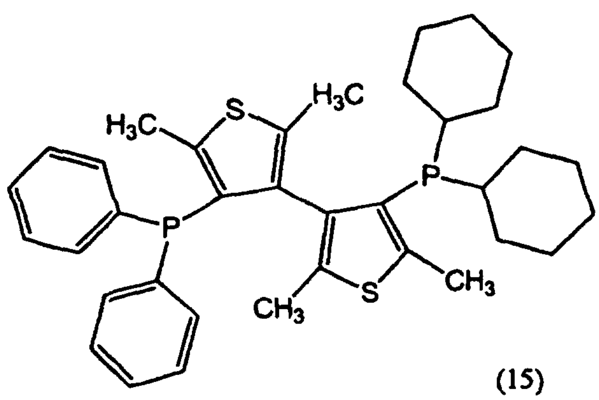

(15)

**12.** Procedure for the preparation of an atropo-isomeric phosphorated ligand of formula (I) having $C_1$ symmetry, as defined in claim 1, comprising one of the following procedure:

A) coupling reaction between aromatic or hetero-aromatic halides with organometallic aryl or hetero-aryl reactants selected from organolithium, organomagnesium, organozinc, and organoboron, in the presence of

catalytic quantities of salts or complexes of copper, nickel, or palladium; or
B) cyclisation and aromatisation, with formation of one of the two heterocyclic rings comprised in the structure of formula (I), of a precursor already containing the other heterocyclic or carbocyclic system ;

in said procedure the introduction of the groups containing the phosphorous atom preceding or following the reaction of formation of the inter-annular bond.

**13.** The procedure according to claim 12, wherein said introduction of the groups containing the phosphorous atom is carried out according one of the following reactions:

in the case of phosphine derivatives :

$$Ar\text{-}[M] + XP(R_1)_2 \rightarrow Ar\text{-}P(R_1)_2$$

$$Ar\text{-}[M] + XP(=O)\,(R_1)_2 \rightarrow Ar\text{-}P(=O)(R_1)_2 \rightarrow Ar\text{-}P(R_1)_2$$

$$Ar\text{-}[M] + (R_2O)_2P(=O)(R_1) \rightarrow Ar_2\text{-}P(=O)(R_1) \rightarrow Ar_2\text{-}PR_1$$

$$Ar\text{-}X + ZP(R_1)_2 \rightarrow Ar\text{-}P(R_1)_2$$

wherein
Ar is an aromatic residue comprised in the structure of formula (I) ;
[M] is an organometallic group ;
X is a halogen ;
Z is an alkaline metal;
$R_1$ and $R_2$ are alkyl or aryl residues ;

- in the case of phosphite or aminophosphine derivatives :

$$Ar\text{-}OH + XP(R_1)_2 \rightarrow Ar\text{-}OP(R_1)_2$$

$$Ind\text{-}NZ + XP(R_1)_2 \rightarrow Ind\text{-}NP(R_1)_2$$

$$Ind\text{-}NZ + XP(=O)(R_1)_2 \rightarrow Ind\text{-}NP(=O)(R_1)_2 \rightarrow Ind\text{-}NP(R_1)_2$$

$$Ar\text{-}NHR_2 + XP(R_1)_2 \rightarrow Ar\text{-}NR_2P(R_1)_2$$

$$Ar\text{-}X + ZOP(R_1)_2 \rightarrow Ar\text{-}OP(R_1)_2$$

Ar is a carbocyclic aromatic or hetero-aromatic residue comprised in the structure of formula (I) ;
Ind is an indole residue ;
X is a halogen;
Z is an alkaline metal;
$R_1$ is an alkyl or aryl group ;
$R_2$ is H or an alkyl or aryl group.

**14.** The procedure according to claim 12, further comprising the resolution of a ligand of formula (I) into its optical antipodes, via separation on chromatographic column or through a membrane, using a chiral stationary substrate or a chiral eluent, or via fractioned crystallisation of a corresponding diastereo-isomeric adduct.

**15.** The procedure according to claim 14, wherein, if the ligand of formula (I) comprises basic or acidic groups, the diastereo-isomeric adduct is the corresponding salt with an entantiomerically pure chiral acid or base; alternatively, the said adduct is the diastereo-isomeric salt between an enantiomerically pure chiral acid and the phosphinoxide corresponding to the present phosphorated ligand. In this case, the optical resolution is followed by reduction of optically active phosphinoxides into phosphines, via treatment with a reducing agent.

**16.** An organometallic complex, comprising a chiral phosphorated ligand of formula (I) as defined in each of the claims from 1 to 11, in the enanatiomerically pure or enriched form, and a transition metal.

**17.** The organometallic complex according to claim 16, wherein the transition metal is selected from the group consisting of Rh, Ru, Ir, Pt, Pd and Ni.

**18.** Use of an organometallic complex according to claim 16 for the preparation of an optically active chiral catalyst.

**19.** Procedure for the preparation of an organic compound in the form of stereoisomer, comprising at least one stereoselective reaction conducted in the presence of at least one organometallic complex as defined in claim 16.

**20.** The procedure according to claim 19, wherein said stereoselective reaction is selected from the group consisting of enantio- and/or diastereoselective reactions of reduction, hydroformylation, hydroboration, hydrosilylation, hydrocyanation, allylation, vinylation and other reactions of formation of the C-C bond.

**Patentansprüche**

**1.** Atropisomerischer chiraler phosphorierter Ligand der Formel (I) mit $C_1$-Symmetrie in der optisch aktiven Form oder in der razemischen Form

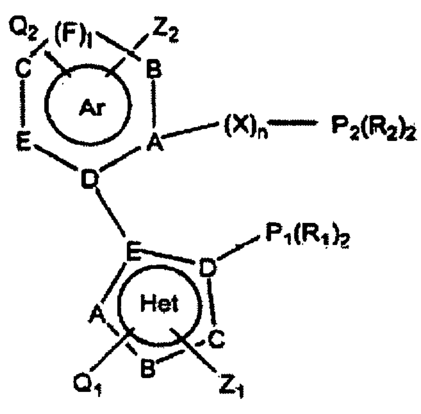

(I)

bei welchem
die Atome A, B, C, D, E und F, die einander gleich oder untereinander verschieden sind, Kohlenstoffatome oder Hetero-Atome sind, die unter Sauerstoff, Stickstoff und Schwefel ausgewählt werden und die zusammen einen aromatischen Ar- oder Het-Rest bilden, in welchem Ar zwischen einem fünfatomigen heterozyklischen Rest und Phenyl gewählt wird und Het ein fünfatomiger heterozyklischer Rest ist und in welchem der besagte fünfatomige heterozyklische aromatische Rest 1 oder 2 Hetero-Atome enthält, die einander gleich oder untereinander verschieden sind und die aus der Gruppe ausgewählt werden, die aus -O-, -S- und $-NR_3-$ besteht, wobei $R_3$ = H, eine Alkylgruppe, eine aromatische Gruppe, eine $(-P_1(R_1)_2)$-Gruppe oder ein Stickstoffatom ist, welches als Hetero-Atom in dem anderen fünfatomigen heterozyklischen Rest enthalten ist, der zur Struktur der Formel (I) gehört;
I = 0, 1; wenn I = 1, dann ist F ein Kohlenstoffatom;
$R_1$ und $R_2$, die an die Phosphoratome gebunden sind und die einander gleich oder untereinander verschieden sind, aus einer linearen, verzweigten oder zyklischen $C_3$-$C_{10}$-Alkylgruppe, einer karbozyklischen aromatischen Gruppe und einer heterozyklischen aromatischen Gruppe mit 5 - 6 Ringgliedern in der zyklischen Verbindung gewählt werden , welche ein oder mehrere Hetero-Atome enthält, die unter Sauerstoff, Schwefel und Stickstoff ausgewählt werden, wobei die besagte karbozyklische oder heterozyklische aromatische Gruppe wahlweise durch

eine oder mehrere Gruppen ersetzt wird, die unter einer linearen oder verzweigten $C_1$-$C_{10}$-Alkylgruppe, einer linearen oder verzweigten $C_1$-$C_{10}$-Alkoxylgruppe, einem Halogen, -$COOR_4$, -$SO_3R_4$ und -$NR_5R_6$ ausgewählt werden, wobei
$R_4$ unter H, Alkyl, Aryl,. Alkali- oder Erdalkalimatall, -$NH_4^+$ und Alkylammonium-Kation mit 4 bis 20 Kohlenstoffatomen und wobei $R_5$ und $R_6$, die einander gleich oder untereinander verschieden sind, H oder Alkyl sind; oder
$R_1$ und $R_2$ zusammen mit dem Phosphoratom eine heterozyklische Verbindung bilden, die 3 bis 6 Atome in der zyklischen Verbindung enthält, die wahlweise durch lineare oder verzweigte $C_1$-$C_{10}$-Alkylgruppen ersetzt sind;
X eine Gruppe (-O-)-Gruppe oder eine (-N($R_7$)-)Gruppe ist, bei welcher $R_7$ unter H, Alkyl und Phenyl gewählt wird;
n gleich 0 oder 1 ist, wenn Ar ein heterozyklischer aromatischer Rest ist;
n gleich 1 ist, wenn Ar Phenyl ist;
$Q_1$, $Q_2$, $Z_1$ und $Z_2$, die einander gleich oder untereinander verschieden sind, aus der Gruppe ausgewählt werden, die aus H, linearer, verzweigter oder zyklischem $C_1$-$C_{10}$-Alkyl, linearem oder verzweigtem $C_1$-$C_{10}$-Alkoxyl, Phenyl und Halogen besteht, oder
Q1 zusammen mit Z1 genommen oder $Q_2$ zusammen mit $Z_2$ genommen einen karbozyklischen aromatischen Ring bilden, der aus Phenyl und Naphthyl ausgewählt wird, wobei der besagte karbozyklische aromatische Ring wahlweise durch eine oder mehrere T-Gruppen ersetzt wird , wobei T unter $C_1$-$C_{10}$-Alkyl, $C_1$-$C_{10}$-Alkoxyl, -$COOR_4$, -$SO_3R_4$ und -$NR_5R_6$ gewählt wird, wobei $R_4$ unter H, $C_1$-$C_{10}$-Alkyl, Phenyl, Alkali- oder Erdalkalimetall, -$NH_4^+$ oder $C_4$-$C_{12}$-Alkylammonium-Kation ausgewählt wird und $R_5$ und $R_6$, die einander gleich oder untereinander verschieden sind, unter H und $C_1$-$C_{10}$-Alkyl ausgewählt werden;
und bei welchem
-$P_1(R_1)R_2$ und -$(X)_n$-$P_2$-$(R_2)_2$ an den entsprechenden karbozyklischen oder heterozyklischen aromatischen Rest mittels eines Kohlenstoffatoms des besagten aromatischen Rests oder mittels eines Stickstoffatoms, das als Hetero-Atom in einem fünfatomigen heterozyklischen Rest enthalten ist, gebunden ist;
wobei der besagte phosphorierte Ligand ferner aufweist:

i) einen Unterschied zwischen den Restladungen der Phosphoratome

$$\Delta Q(P) = Q(P_1) - Q(P_2) > 0,05,$$

wobei $Q(P_1)$ und $Q(P_2)$ die Werte des Unterschieds zwischen der Anzahl der Valenzelektronen und der Anzahl der Elektronen ist, die für die Phosphoratome $P_1$ und $P_2$ tatsächlich vorhanden sind,
ii) einen Kegelwinkel $\beta_n$ ("natürlicher Bisswinkel" nach Casey), der von 80° bis 130° reicht und als bevorzugter Chelationswinkel $P_1$-M-$P_2$ zwischen den Phosphoratomen $P_1$ und P2 und einem Übergangsmetall M definiert ist;
iii) einen energetischen Schwellwert der gegenseitigen Umwandlung zwischen den zwei Spiegelbildisomeren eines gegebenen Liganden

$$\Delta E = E_{trans} - E_{min} \geq 28 \text{ kcal/mol},$$

wobei $E_{trans}$ der Energiewert für den Übergangszustand ist und $E_{min}$ derjenige Wert ist, der mit dem Zustand minimaler Energie der Spiegelbildisomere im Zusammenhang steht, der in kcal/mol ausgedrückt wird.

**2.** Phosphorierter Ligand nach Anspruch 1, bei welchem

i) der besagte Unterschied $\Delta Q(P) = Q(P_1) - Q(P_2) > 0,15$;
ii) der besagte "natürliche Bisswinkel" $\beta_n$ zwischen 83° und 120° liegt.

**3.** Phosphorierter Ligand nach Anspruch 1, bei welchem der besagte phosphorierte Ligand zwischen einem Liganden der Formel (I)a und einem Liganden der Formel (I)b gewählt wird:

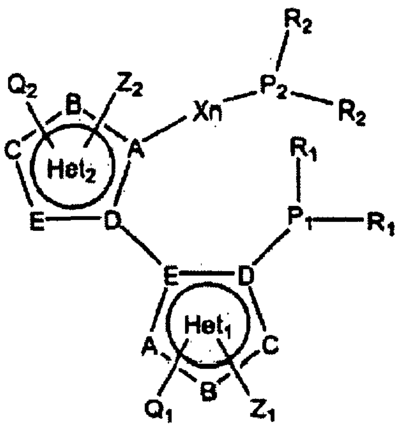

(I)a

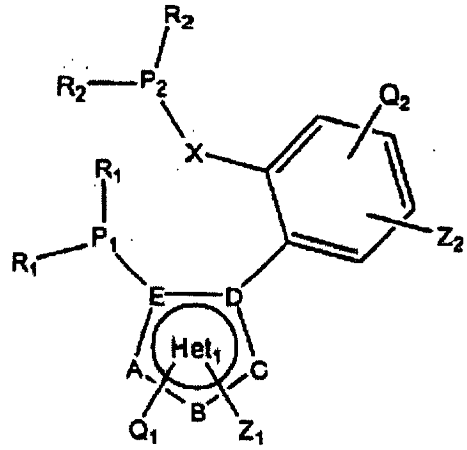

(I)b

wobei $Het_1$ und $Het_2$ fünfatomige heterozyklische aromatische Ringe sind, die einander gleich oder untereinander verschieden sind und die als Het im Anspruch 1 festgelegt sind;
n gleich 0 oder 1 ist;
X, A, B, C, D, E, $Q_1$, $Q_2$, $Z_1$ und $Z_2$ so sind, wie sie im Anspruch 1 festgelegt sind.

**4.** Phosphorierter Ligand nach Anspruch 1, bei welchem der besagte heterozyklische. Rest aus der Gruppe, die aus Thiophen, Pyrrol, Furan, Imidazol, Isoxazol, Isothiazol, Pyrazol und Triazol besteht, ausgewählt wird .

**5.** Phosphorierter Ligand nach Anspruch 1, bei welchem $Q_1$ zusammen genommen mit $Z_1$ oder $Q_2$ zusammen genommen mit $Z_2$ einen karbozyklischen Ring bilden und Het mit Phenyl oder Naphthyl kondensiert wird.

**6.** Phosphorierter Ligand nach Anspruch 5, bei welchem der besagte heterozyklische, mit Phenyl kondensierte Ring Het aus der Gruppe ausgewählt wird, die aus Benzothiophen, Naphthothiophen, Indol, Benzofuran und Benzoimidazol besteht.

**7.** Phosphorierter Ligand nach Anspruch 1, bei welchem der besagte phosphorierte Ligand unter einem Liganden der Formel (I)c, (I)d und (I)e ausgewählt wird:

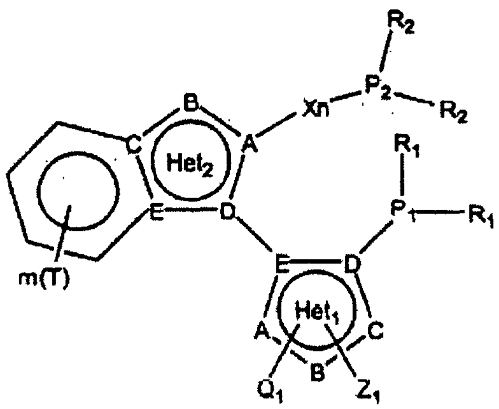

(I)c

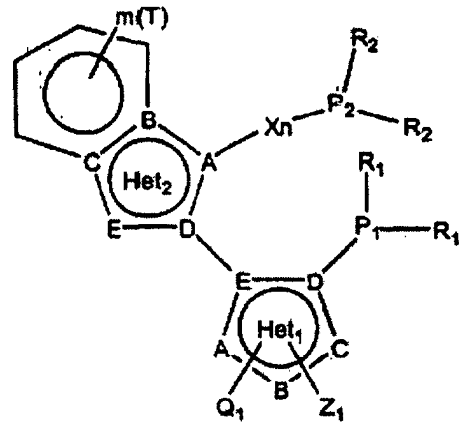

(I)d

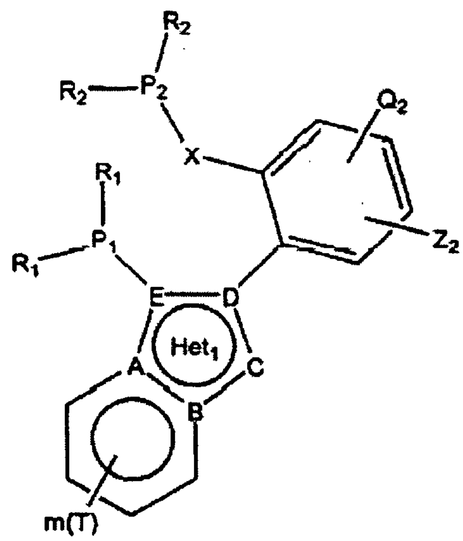

(I)e

wobei $Het_1$ und $Het_2$ so festgelegt sind wie Het im Anspruch 1;
A, B, C, D, E, $Q_1$, $Z_1$, $P_1$, $R_1$, $Q_2$, $Z_2$, $P_2$, $R_2$ und T so definiert sind wie im Anspruch 1 für die Formel (I);
m gleich 0, 1 oder 2 ist.

**8.** Phosphorierter Ligand nach Anspruch 1, bei welchem der besagte heterozyklische aromatische Rest aus der Gruppe ausgewählt wird, die aus 2,5-Dimethyl-thien-3-yl, 4,6-Dimethyl-benzofur-3-yl, 3-Methyl-indol-2-yl, 1-N-Methyl-indol-2-yl und Benzothien-3-yl besteht; und der besagte karbozyklische aromatische Rest Phenyl ist.

**9.** Phosphorierter Ligand nach Anspruch 1, bei welchem die besagten Gruppen $-P_1(R_1)_2$ und $-P_2(R_2)_2$ unter Diphenylphosphin, Dizyklohexylphosphin, $J_1$, $J_2$ und $J_3$ ausgewählt werden, wobei $J_1$, $J_2$ und $J_3$ die folgenden Formeln haben:

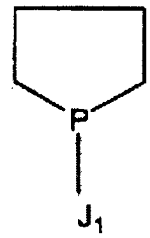

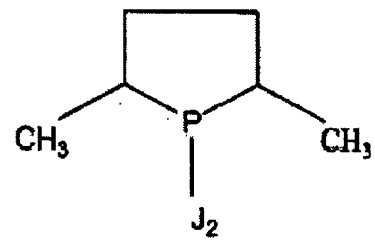

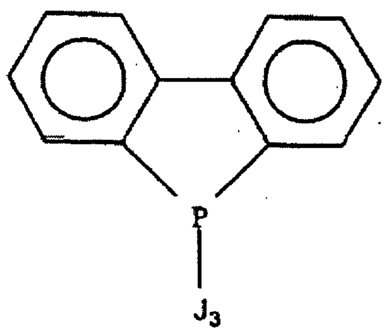

10. Phosphorierter Ligand nach Anspruch 1, welcher eine der folgenden Substrukturen enthält: (4-Diphenylphosphin)- oder (4-Dizyklohexylphosphin)-2,5-dimethylthien-3-yl; (1-N-diphenylphosphin)- oder (1-N-Dizyklohexylphosphin)-3-methylindol-2-yl; (3-Diphenylphosphin)- oder (2-Dizxklohexylphosphin)-1-N-methylindol-2-yl; 2-(Diphenylphosphin)-, oder 2-(Dizyklohexylphosphin)-benzothien-3-yl; 2-(Diphenylphosphin-oxy)- oder 2-(Dizyklohexylphosphin-oxy)-phenyl-1-yl; 4-(Diphenylphosphin-oxy)- oder 4-(Dizyklohexylphosphin-oxy)-2,5-dimethyl-thien-3-yl; 1-N-(2',5'-Dimethylphospholyl)- oder 1-N-(Dibenzophospholyl)-3-methyl-indol-2-yl.

11. Phosphorierter Ligand nach Anspruch 1, bei welchem der besagte phosphorierte Ligand aus den Verbindungen von (1) bis (15) gewählt wird.

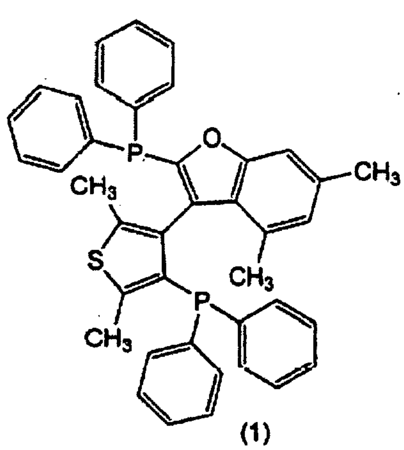

(1)

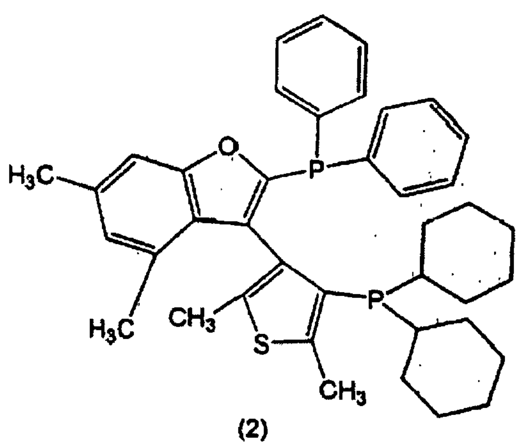

(2)

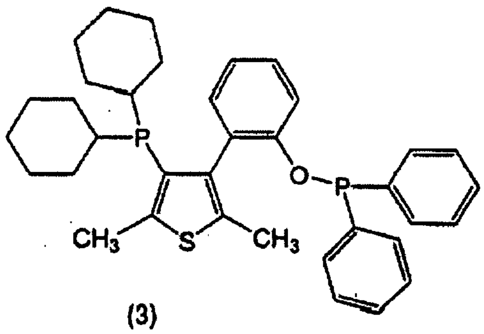

(3)

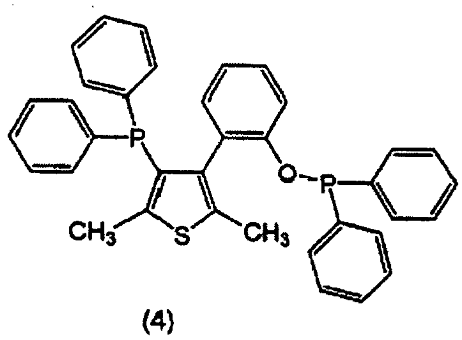

(4)

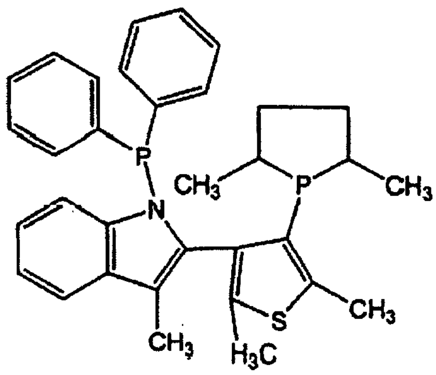

(5)

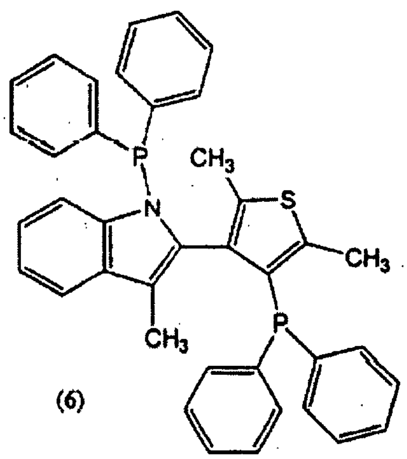

(6)

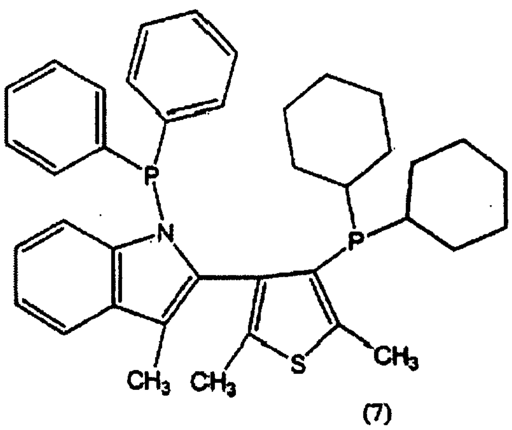

(7)

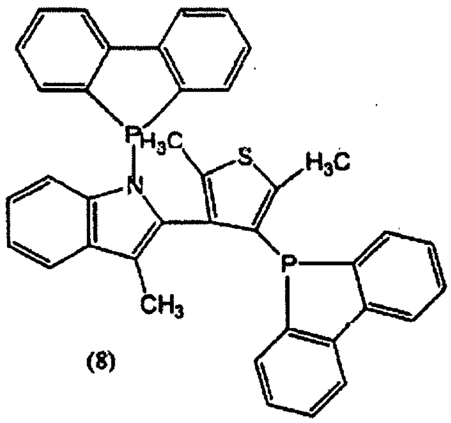

(8)

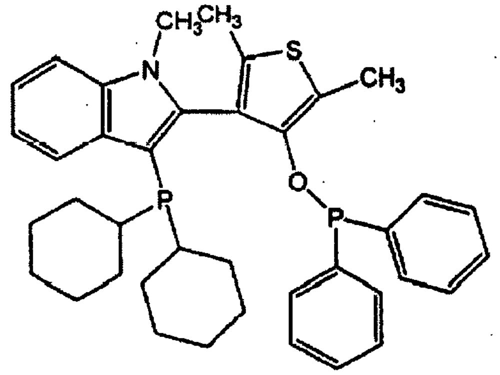

(9)

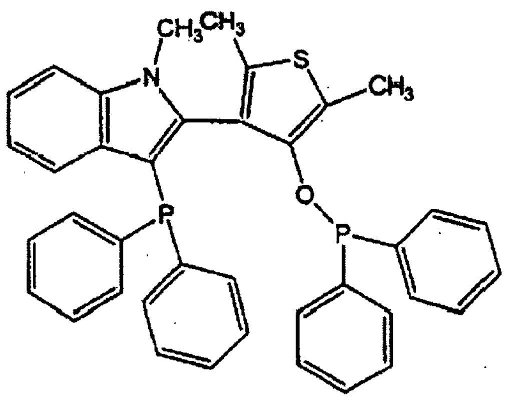

(10)

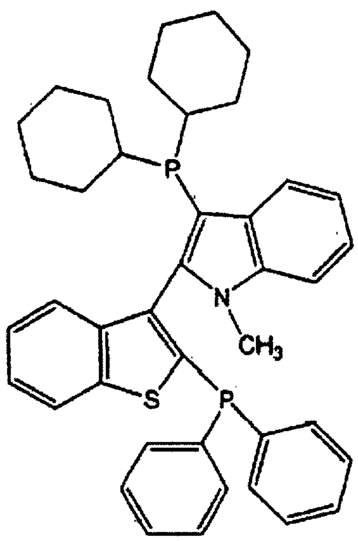

(11)

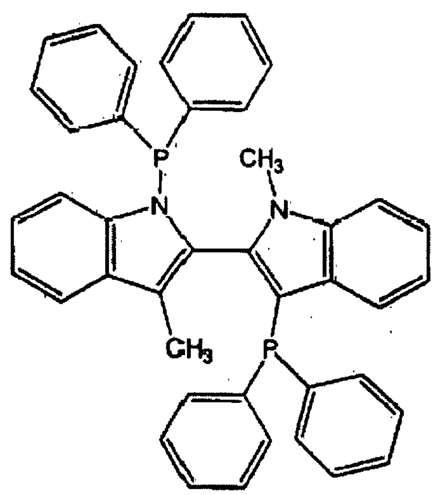

(12)

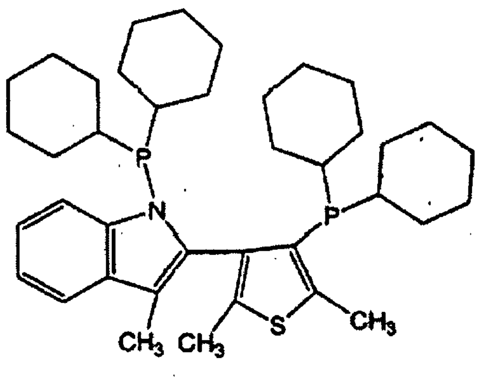

(13)

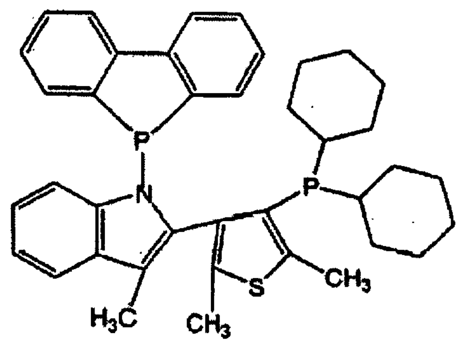

(14)

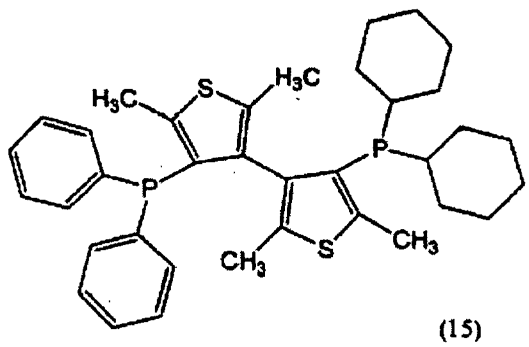

(15)

**12.** Verfahren zur Herstellung eines atropisomerischen phosphorierten Liganden der Formel (I) mit C1-Symmetrie, wie er in Anspruch 1 festgelegt ist, wobei dieses Verfahren eines der folgenden Verfahren umfasst:

A) eine Kopplungsreaktion zwischen aromatischen oder hetero-aromatischen Haliden mit organometallischen Aryl- oder Hetero-Aryl-Reaktanten, die unter Organolithium, Organomagnesium, Organozink und Organobor ausgewählt werden, in Gegenwart von katalytischen Mengen an Salzen oder Komplexen von Kupfer, Nickel oder Palladium; oder
B) Ringschluss und Aromatisierung eines Vorläufers, der bereits das andere heterozyklische oder karbozyklische System enthält, unter Bildung von einem der zwei heterozyklischen Ringe, die in der Struktur von Formel (I) enthalten sind;

wobei in dem besagten Verfahren die Einführung der Gruppen, welche das phosphorierte Atom enthalten, der Reaktion der Bildung der Zwischenringbindung vorausgeht oder ihr folgt.

**13.** Verfahren nach Anspruch 12, bei welchem die besagte Einführung der Gruppen, welche das phosphorierte Atom enthalten, gemäß einer der folgenden Reaktionen ausgeführt wird:

Im Fall der Phosphin-Derivate:

$$\text{Ar-[M]} + \text{XP(R}_1)_2 \rightarrow \text{Ar-P(R}_1)_2$$

$$\text{Ar-[M]} + \text{XP(=O) (R}_1)_2 \rightarrow \text{Ar-P(=0)(R}_1)_2 \rightarrow \text{Ar-P(R}_1)_2$$

$$\text{Ar-[M]} + (\text{R}_2\text{O})_2\text{P(=0)(R}_1) \rightarrow \text{Ar}_2\text{-P(=0)(R}_1) \rightarrow \text{Ar}_2\text{-PR}_1$$

$$Ar\text{-}X + ZP(R1)_2) \rightarrow Ar\text{-}P(R_1)_2$$

wobei
Ar ein aromatischer Rest ist, der in der Struktur der Formel (I) enthalten ist;
[M] eine organometallische Gruppe ist;
X ein Halogen ist;
Z ein Alkalimetall ist;
$R_1$ und $R_2$ Alkyl- oder Arylreste sind;

- im Fall von Phosphit- oder Aminophosphinderivaten:

$$Ar\text{-}OH + XP(R_1)_2 \rightarrow Ar\text{-}OP(R_1)_2$$

$$Ind\text{-}NZ + XP(R_1)_2 \rightarrow Ind\text{-}NP(R_1)_2$$

$$Ind\text{-}NZ + XP(=0)(R_1)_2 \rightarrow Ind\text{-}NP(=0)(R_1)_2 \rightarrow Ind\text{-}NP(R_1)_2$$

$$Ar\text{-}NHR_2 + XP(R_1)_2 \rightarrow Ar\text{-}NR_2P(R_1)_2$$

$$Ar\text{-}X + ZOP(R_1)_2 \rightarrow Ar\text{-}OP(R_1)_2$$

Ar ein karbozyklischer aromatischer oder heterp-aromatischer Rest ist, der in der Struktur der Formel (I) enthalten ist;
Ind ein Indol-Rest ist;
X ein Halogen ist ;
Z ein Alkalimetall ist;
R1 eine Alkyl- oder Arylgruppe ist;
R2 H oder eine Alkyl- oder Arylgruppe ist.

**14.** Verfahren nach Anspruch 12, welches ferner die Auflösung eines Liganden der Formel (I) in seine optischen Antipoden über die Trennung auf einer chromatografischen Säule oder durch eine Membrane unter Verwendung eines chiralen stationären Substrats oder eines chiralen Eluationsmittels oder über fraktionierte Kristallisierung eines entsprechenden diastereomeren Addukts umfasst.

**15.** Verfahren nach Anspruch 14, bei welchem, wenn der Ligand der Formel (I) basische oder saure Gruppen aufweist, das diastereomere Addukt das entsprechende Salz mit einer spiegelbildlich reinen chiralen Säure oder Base ist; alternativ ist das besagte Addukt das diastereomere Salz zwischen einer spiegelbildlich reinen chiralen Säure und dem Phosphinoxid, weelches dem vorliegenden phosphorierten Liganden entspricht. In diesem Fall folgt der optischen Auflösung die Reduktion der optisch aktiven Phosphinoxide in Phosphine über die Behandlung mit einem Reduktionsmittel.

**16.** Organometallischer Komplex, welcher einen chiralen phosphorierten Liganden der Formel (I), wie er in jedem der Ansprüche von 1 bis 11 festgelegt ist, in der spiegelbildlich reinen oder angereicherten Form und ein Übergangsmetall enthält.

**17.** Organometallischer Komplex nach Anspruch 16, bei welchem das Übergangsmetall aus der Gruppe ausgewählt wird, die aus Rh, Ru, Ir, Pt, Pd und Ni besteht.

**18.** Verwendung eines organometallischen Komplexes gemäß Anspruch 16 für die Herstellung eines optisch aktiven chiralen Katalysators.

**19.** Verfahren zur Herstellung einer organischen Verbindung in Form eines Stereoisomers, welches wenigstens eine

stereoselektive Reaktion umfasst, die in Gegenwart von mindestens einem organometallischen Komplex, wie er im Anspruch 16 festgelegt ist, durchgeführt wird.

**20.** Verfahren nach Anspruch 19, bei welchem die besagte stereoselektive Reaktion aus der Gruppe ausgewählt wird, welche aus den spiegelbildlichen und/oder diastereoselektiven Reaktionen der Reduktion, Hydroformylierung, Hydroborierung, Hydrosilylierung, Hydrocyanierung, Allylierung, Vinylierung und anderen Reaktionen zur Bildung der Bindung C-C besteht.

## Revendications

**1.** Ligand phosphoré chiral atropo-isomérique de formule

(I) ayant une symétrie $C_1$, sous forme optiquement active ou sous forme racémique

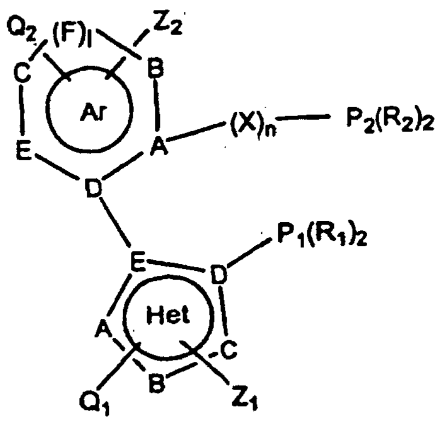

(I)

dans laquelle

- les atomes A, B, C, D, E et F identiques ou différents les uns des autres sont des atomes de carbone ou des hétéroatomes choisis parmi l'oxygène, l'azote et le soufre, qui forment en association un résidu Ar ou Het aromatique, où Ar est choisi entre un résidu hétérocyclique penta-atomique et le groupe phényle, et Het est un résidu hétérocyclique penta-atomique, et où ledit résidu aromatique hétérocyclique penta-atomique contient 1 ou 2 hétéroatomes identiques ou différents l'un de l'autre, choisis au sein du groupe consistant en -O-, -S- et $-NR_3-$ dans lequel $R_3$ = H, un groupe alkyle, un groupe aromatique, un groupe $-P_1(R_1)_2$ ou un atome d'azote compris en tant qu'hétéroatome dans l'autre résidu hétérocyclique penta-atomique appartenant à la structure de formule (I) ;
- I = 0,1 ; lorsque I = 1, F est un atome de carbone ;
- $R_1$ et $R_2$ liés aux atomes de phosphore, identiques ou différents l'un de l'autre, sont choisis entre un groupe alkyle $C_3$-$C_{10}$ linéaire, ramifié ou cyclique, un groupe aromatique carbocyclique et un groupe aromatique hétérocyclique ayant 5-6 atomes dans le cycle, contenant un ou plusieurs hétéroatomes choisis parmi l'oxygène, le soufre et l'azote, où ledit groupe carbocyclique ou hétérocyclique aromatique est éventuellement substitué par un ou plusieurs groupes choisis entre un groupe alkyle $C_1$-$C_{10}$ linéaire ou ramifié, un groupe alcoxyle $C_1$-$C_{10}$ linéaire ou ramifié, un halogène, $-COOR_4$, $-SO_3R_4$ et $-NR_5R_6$ où $R_4$ est choisi parmi H, les groupes alkyle, aryle, les métaux alcalins ou alcalinoterreux, $-NH_4^+$ et les cations alkylammonium ayant 4 à 20 atomes de carbone ; et où $R_5$ et $R_6$, identiques ou différents l'un de l'autre représentent H ou un groupe alkyle ; ou
- $R_1$ et $R_2$ en association avec l'atome de phosphore, forment un hétérocycle ayant 3 à 6 atomes dans le cycle, éventuellement substitué par des groupes alkyle $C_1$-$C_{10}$ linaires ou ramifiés ;
- X représente un groupe -O- ou un groupe $-N(R_7)-$ dans lequel $R_7$ est choisi parmi H, les groupes alkyle et phényle ;
- n est égal à 0 ou à 1 lorsque Ar est un résidu aromatique hétérocyclique ;
- n est égal à 1 lorsque Ar est un groupe phényle ;
- $Q_1$, $Q_2$, $Z_1$ et $Z_2$, identiques ou différents les uns des autres, sont choisis au sein du groupe consistant en H,

en des groupes alkyle $C_1$-$C_{10}$ linéaires, ramifiés ou cycliques, en des groupes alcoxyle $C_1$-$C_{10}$ linéaires, ramifiés ou cycliques, en un groupe phényle et en groupe halogène, ou

- $Q_1$ pris en association avec $Z_1$, ou $Q_2$ pris en association avec $Z_2$, forme un cycle aromatique carbocyclique choisi entre les groupes phényle et naphtyle, ledit cycle aromatique carbocyclique étant éventuellement substitué par un ou plusieurs groupes T, où T est choisi parmi les halogènes, les groupe alkyle $C_1$-$C_{10}$, alcoxyle $C_1$-$C_{10}$, $-COOR_4$, $-SO_3R_4$ et $-NR_5R_6$ où $R_4$ est choisi parmi H, les groupes alkyle $C_1$-$C_{10}$, phényle, les métaux alcalins ou alcalinoterreux, $-NH_4^+$ ou les cations alkylammonium $C_4$-$C_{12}$ et où $R_5$ et $R_6$, identiques ou différents l'un de l'autre, sont choisis entre H et les groupes alkyle $C_1$-$C_{10}$ ; et dans laquelle
- $P_1(R_1)_2$ et $-(X)_n-P_2(R_2)_2$ sont liés au résidu aromatique carbocyclique ou hétérocyclique correspondant au moyen d'un atome de carbone dudit résidu aromatique ou au moyen d'un atome d'azote compris en tant qu'hétéroatome dans un résidu hétérocyclique penta-atomique ;

ledit ligand phosphoré ayant aussi :

(i) une différence entre les charges résiduelles des atomes de phosphore

$$\Delta Q(P) = Q(P_1) - Q(P_2) > 0,05$$

où $Q(P_1)$ et $Q(P_2)$ sont les valeurs de la différence entre le nombre d'électrons de valence et le nombre d'électrons réellement présents pour les atomes de phosphore $P_1$ et $P_2$ ;
(ii) un angle conique $\beta_n$ (« natural bite angle » selon Casey) compris entre 80° et 130° défini comme étant l'angle de chélation préféré $P_1$-M- $P_2$ entre les atomes de phosphore $P_1$ et $P_2$ et un métal de transition M ;
(iii) une valeur de barrière d'énergie d'interconversion entre les deux énantiomères d'un ligand donné

$$\Delta E = E_{trans} - E_{min} \geq 28 \text{ kcal/mole}$$

où $E_{trans}$ est la valeur de l'énergie pour l'état de transition et $E_{min}$ est la valeur associée à l'état d'énergie minimum des énantiomères, exprimées en kcal/mole.

**2.** Ligand phosphoré selon la revendication 1, dans lequel

- (i) ladite différence $\Delta Q(P) = Q(P_1) - Q(P_2)$ est > 0,15 ;
- (ii) ledit « natural bite angle » $\beta_n$ est compris entre 83° et 120°.

**3.** Ligand phosphoré selon la revendication 1, dans lequel ledit ligand phosphoré est choisi entre un ligand de formule (I)a et un ligand de formule (I)b :

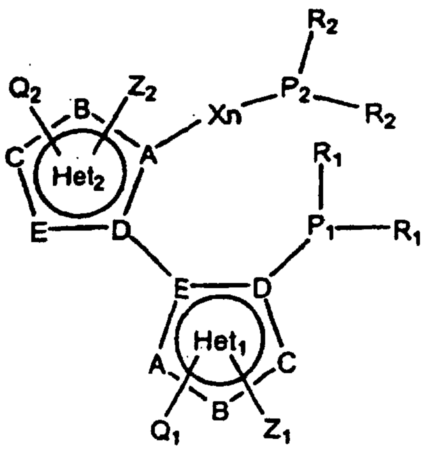

(I)a

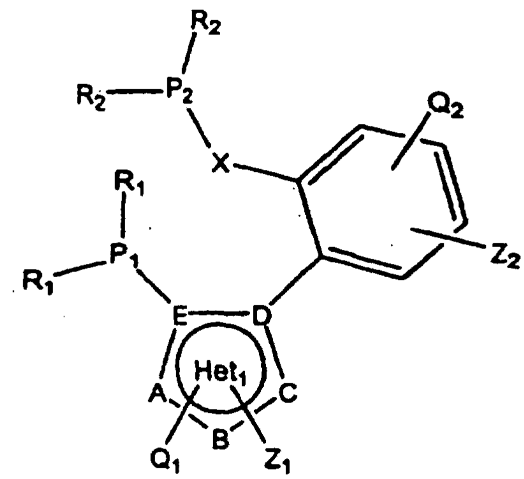

(I)b

dans lesquelles

- $Het_1$ et $Het_2$ représentent des cycles aromatiques hétérocycliques penta-atomiques, identiques ou différents les uns des autres, définis comme Het dans la revendication 1 ;
- n est égal à 0 ou à 1 ;
- X, A, B, C, D, E, $Q_1$, $Q_2$, $Z_1$ et $Z_2$ sont tels que définis dans la revendication 1.

**4.** Ligand phosphoré selon la revendication 1, dans lequel ledit résidu hétérocyclique est choisi au sein du groupe consistant en le thiophène, le pyrrole, le furanne, l'imidazole, l'isoxasole, l'isothiazole, le pyrazole et le triazole.

**5.** Ligand phosphoré selon la revendication 1, dans lequel $Q_1$ pris en association avec $Z_1$, ou $Q_2$ pris en association avec $Z_2$ forme un cycle carbocyclique, et Het est condensé avec un groupe phényle ou naphtyle.

**6.** Ligand phosphoré selon la revendication 5, dans lequel ledit résidu hétérocyclique Het condensé avec un groupe phényle, est choisi au sein du groupe consistant en le benzothiophène, le naphtothiophène, l'indole, le benzofuranne et le benzoimidazole.

**7.** Ligand phosphoré selon la revendication 1, dans lequel ledit ligand phosphoré est choisi entre un ligand de formule (I)c, de formule (I)d et de formule (I)e :

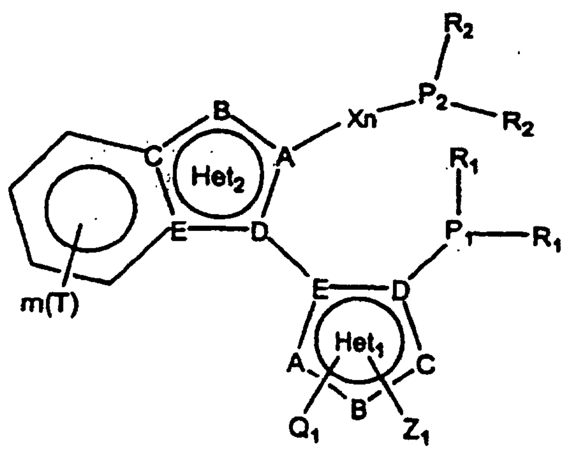

(I)c

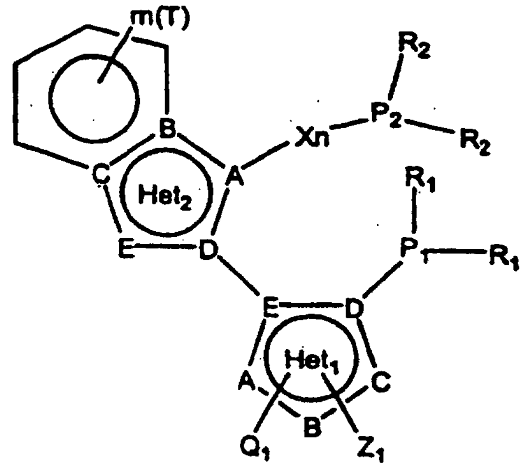

(I)d

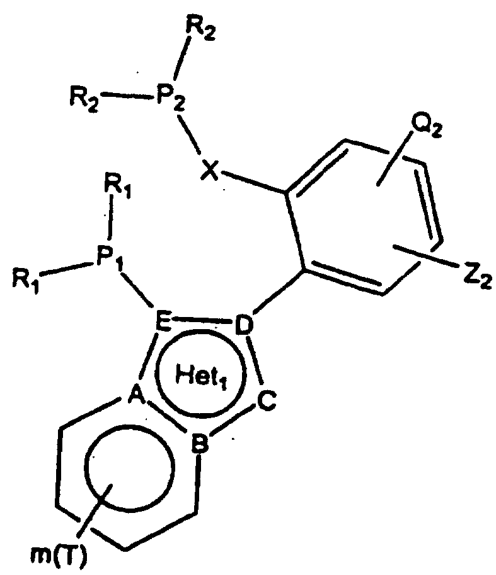

(I)e

dans lesquelles

- $Het_1$ et $Het_2$ sont définis comme Het dans la revendication 1 ;
- A, B, C, D, E, $Q_1$, $Z_1$, $P_1$, $R_1$, $Q_2$, $Z_2$, $P_2$, $R_2$ et T sont tels que définis dans la revendication 1 pour la formule (I) ;
- m est égal à 0, 1 ou 2.

**8.** Ligand phosphoré selon la revendication 1, dans lequel ledit résidu aromatique hétérocyclique est choisi au sein du groupe consistant en le 2,5-diméthylthién-3-yle, le 4,6-diméthylbenzofur-3-yle, le 3-méthylindol-2-yle, le 1-N-méthylindol-2-yle et le benzothién-3-yle ; et ledit résidu aromatique carbocyclique est le groupe phényle.

**9.** Ligand phosphoré selon la revendication 1, dans lequel lesdits groupes $-P_1(R_1)_2$ et $-P_2\ (R_2)_2$ sont choisis parmi

la diphénylphosphine, la dicyclohexylphosphine, $J_1$, $J_2$ et $J_3$, où $J_1$, $J_2$ et $J_3$ ont les formules suivantes :

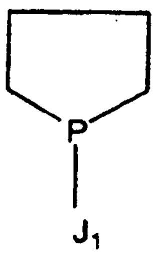

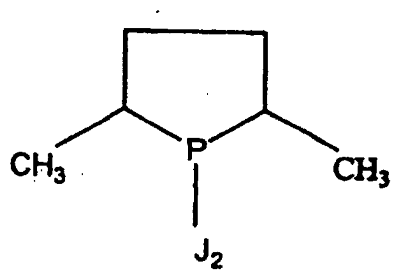

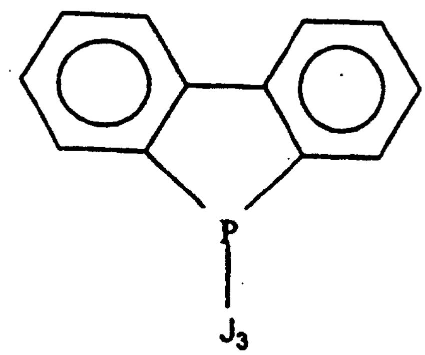

**10.** Ligand phosphoré selon la revendication 1, contenant une des sous-structures suivantes :

- (4-diphénylphosphine)- ou (4-dicyclohexylphosphine)-2,5-diméthyl-thién-3-yle ;
- (1-N-diphénylphosphine)- ou (1-N-dicyclohexylphosphine)-3-méthyl-indol-2-yle ;
- (3-diphénylphosphine)- ou (3-dicyclohexylphosphine)-1-N-méthyl-indol-2-yle ;
- 2-(diphénylphosphine)- ou 2-(dicyclohexylphosphine)benzothién-3-yle ;
- 2-(diphénylphosphine-oxy)- ou 2-(dicyclohexylphosphine-oxy)phényl-1-yle ;
- 4-(diphénylphosphine-oxy)- ou 4-(dicyclohexylphosphine-oxy)-2,5-diméthylthién-3-yle ; .
- 4-(2',5'-diméthylphospholyl)- ou 4-(dibenzophospholyl)-2,5-diméthyl-thién-3-yle ;
- 1-N-(2',5'-diméthylphospholyl)- ou 1-N-(dibenzophospholyl)-3-méthylindol-2-yle.

**11.** Ligand phosphoré selon la revendication 1, dans lequel ledit ligand phosphoré est choisi parmi les composés (1) à (15).

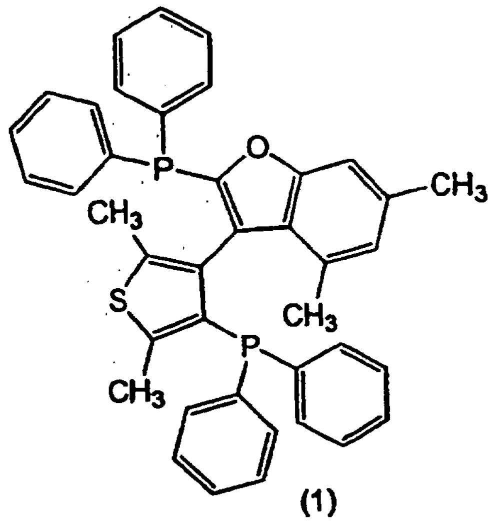

(1)

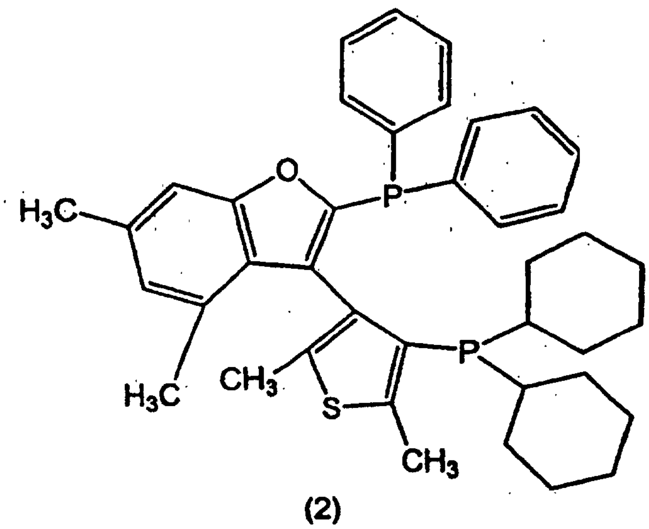

(2)

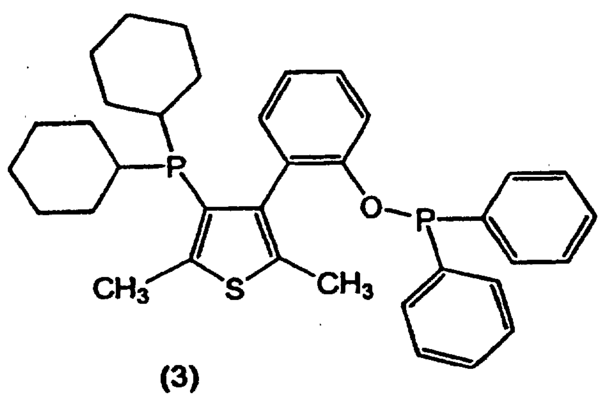

(3)

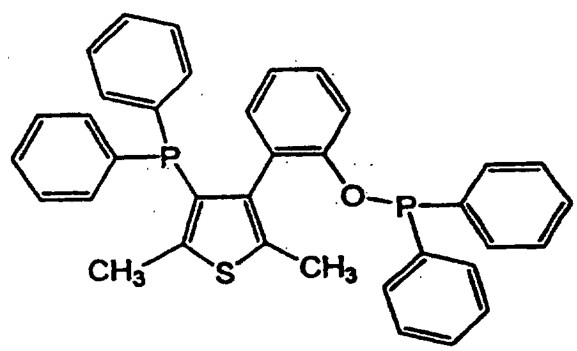

(4)

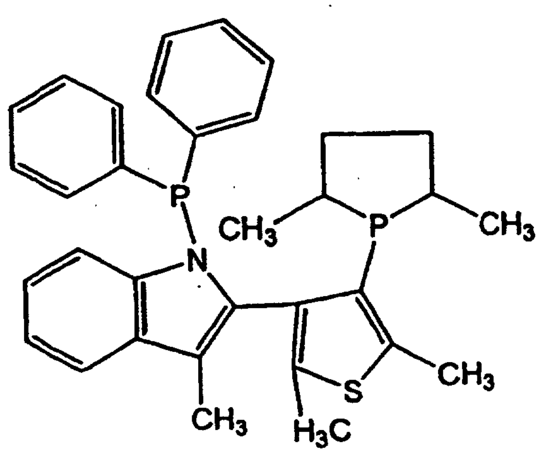

(5)

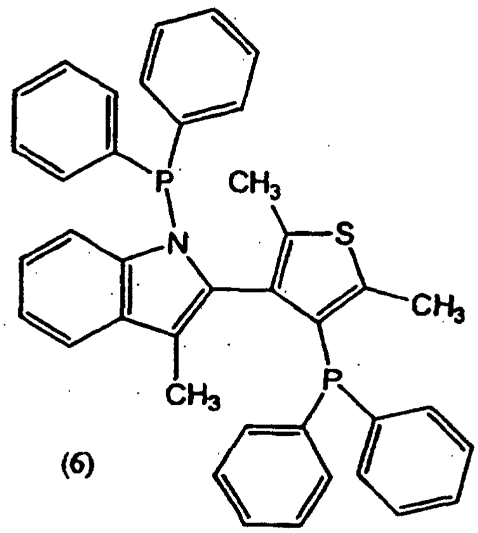

(6)

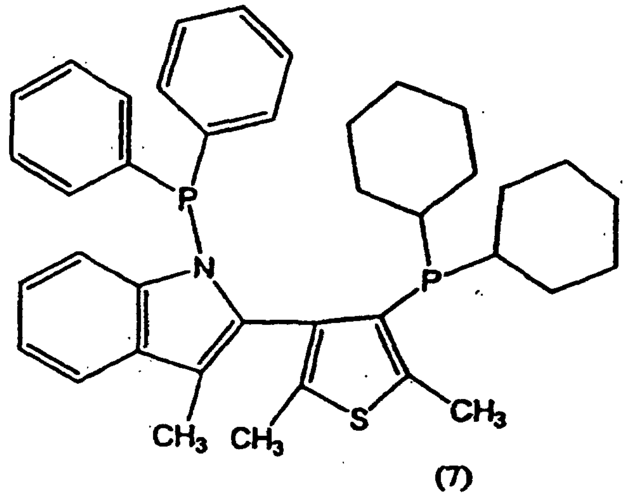

(7)

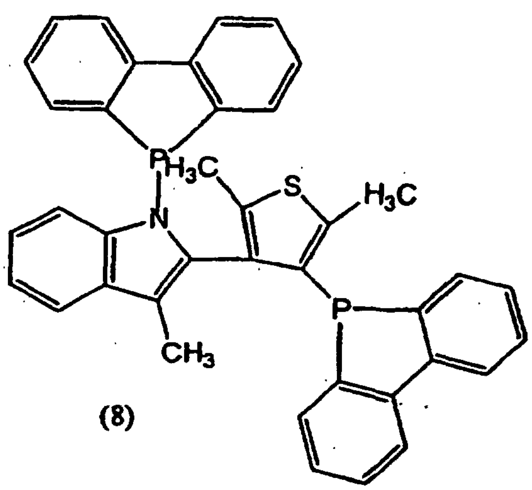

(8)

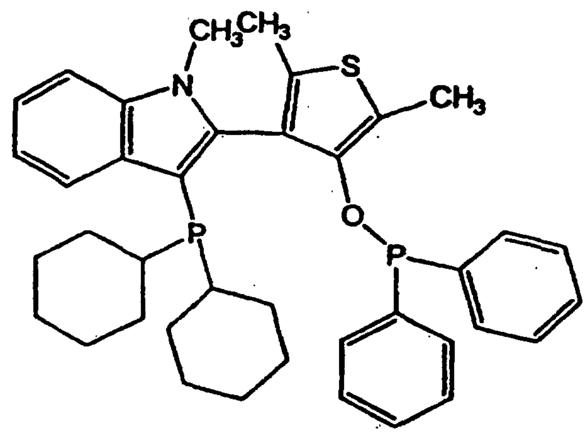

(9)

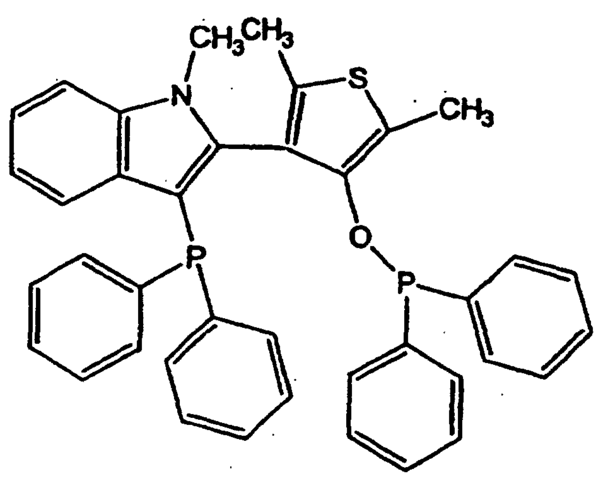

(10)

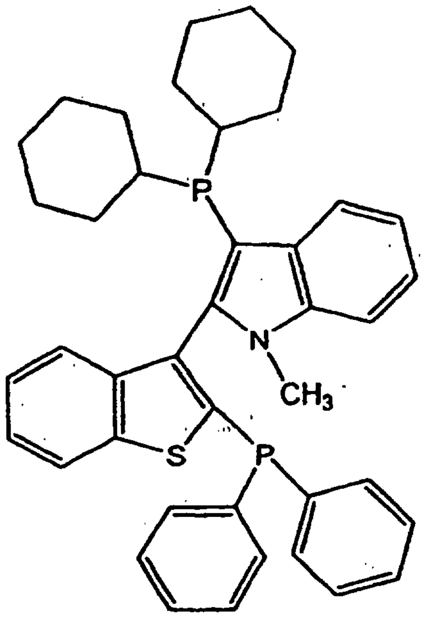

(11)

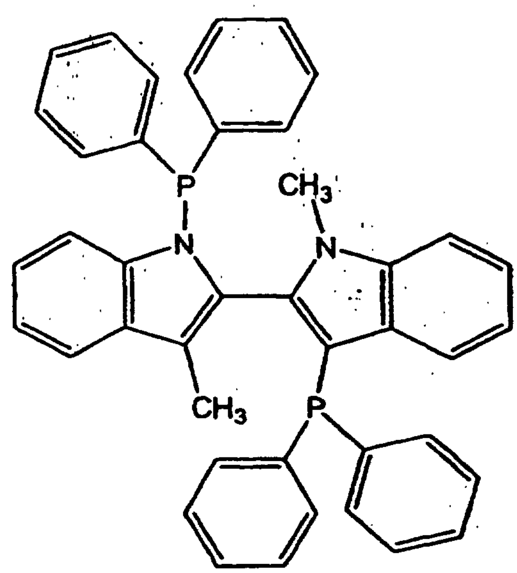

(12)

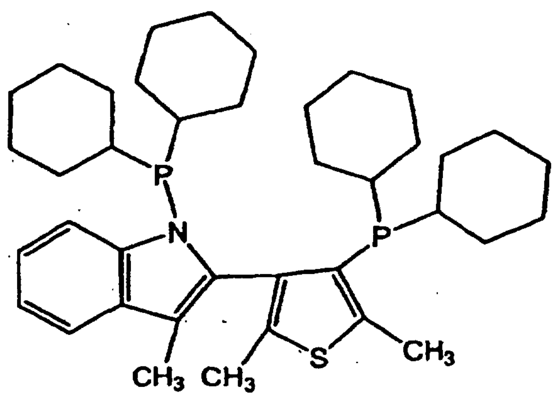

(13)

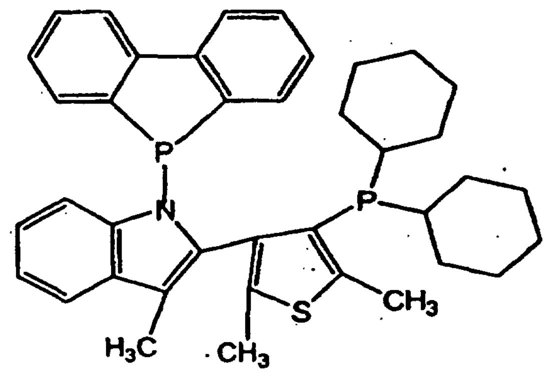

(14)

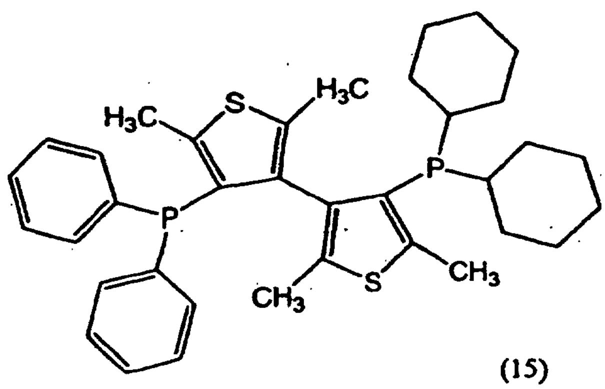

(15)

**12.** Procédé pour la préparation d'un ligand phosphoré atropo-isomérique de formule (I) ayant une symétrie $C_1$ selon la revendication 1, comprenant une des procédures suivantes :

(A) réaction de couplage entre des halogénures aromatiques ou hétéro-aromatiques avec des réactifs organo-métalliques aryle ou hétéro-aryle choisis parmi les organolithiens, les organomagnésiens, les organozinciques et les organoboriques, en présence des quantités catalytiques de sels ou complexes de cuivre, de nickel ou de palladium ; ou

(B) cyclisation et aromatisation, avec formation de l'un des deux cycles hétérocycliques compris dans la structure de formule (I), d'un précurseur contenant déjà l'autre système hétérocyclique ou carbocyclique ;

dans ledit procédé, l'introduction des groupes contenant l'atome de phosphore précédant ou faisant suite à la réaction de formation de la liaison inter-annulaire.

**13.** Procédé selon la revendication 12, dans lequel ladite introduction des groupes contenant l'atome de phosphore est effectuée selon l'une des réactions suivantes :

- dans le cas des dérivés phosphine :

$$Ar\text{-}[M] + XP(R_1)_2 \rightarrow Ar\text{-}P(R_1)_2$$

$$Ar\text{-}[M] + XP(=O)(R_1)_2 \rightarrow Ar\text{-}P(=O)(R_1)_2 \rightarrow Ar\text{-}P(R_1)_2$$

$$Ar\text{-}[M] + (R_2O)_2P(=O)(R_1) \rightarrow Ar_2\text{-}P(=O)(R_1) \rightarrow Ar_2\text{-}PR_1$$

$$Ar\text{-}X + ZP(R_1)_2 \rightarrow Ar\text{-}P(R_1)_2$$

où
Ar est un résidu aromatique compris dans la structure de formule (I) ;
[M] est un groupe organo-métallique ;
X est un halogène, ;
Z est un métal alcalin ;
$R_1$ et $R_2$ sont des résidus alkyle ou aryle ;

- dans le cas des dérivés phosphite ou aminophosphine :

$$Ar\text{-}OH + XP(R_1)_2 \rightarrow Ar\text{-}OP(R_1)_2$$

$$Ind\text{-}NZ + XP(R_1)_2 \rightarrow Ind\text{-}NP(R_1)_2$$

$$Ind\text{-}NZ + XP(=O)(R_1)_2 \rightarrow Ind\text{-}NP(=O)(R_1)_2 \rightarrow Ind\text{-}NP(R_1)_2$$

$$Ar\text{-}NHR_2 + XP(R_1)_2 \rightarrow Ar\text{-}NR_2P(R_1)_2$$

$$Ar\text{-}X + ZOP(R_1)_2 \rightarrow Ar\text{-}OP(R_1)_2$$

où
Ar est un résidu aromatique ou hétéro-aromatique compris dans la structure de formule (I) ;
Ind est un résidu indole ;
X est un halogène, ;
Z est un métal alcalin ;
$R_1$ est un groupe alkyle ou aryle ;
$R_2$ est H ou un groupe alkyle ou aryle.

**14.** Procédé selon la revendication 12, comprenant aussi la décomposition d'un ligand de formule (I) en ses antipodes optiques, via une séparation sur colonne de chromatographie ou à travers une membrane, par l'utilisation d'un substrat chiral stationnaire ou d'un éluant chiral, ou via la cristallisation fractionnée d'un adduit diastéréo-isomérique correspondant.

**15.** Procédé selon la revendication 14, dans lequel, si le ligand de formule (I) comprend des groupes basiques ou acides, l'adduit diastéréoisomérique est le sel correspondant avec un acide ou une base chiral énantiomériquement pur ; en alternative, ledit adduit est le sel diastéréo-isomérique entre un acide chiral énantiomériquement pur et le phosphinoxyde correspondant au présent ligand phosphoré. Dans ce cas, la décomposition optique est suivi de la réduction des phosphinoxydes optiquement actifs en phosphines, par traitement à l'aide d'un agent réducteur.

**16.** Complexe organo-métallique comprenant un ligand phosphoré chiral de formule (I) selon chacune des revendications 1 à 11, sous une forme énantiomériquement pure ou enrichie, et un métal de transition.

**17.** Complexe organo-métallique selon la revendication 16, dans lequel le métal de transition est choisi au sein du groupe consistant en Rh, Ru, Ir, Pt, Pd et Ni.

**18.** Utilisation d'un complexe organo-métallique selon la revendication 16, pour la préparation d'un catalyseur chiral optiquement actif.

**19.** Procédé pour la préparation d'un composé organique sous forme de stéréoisomère, comprenant au moins une réaction stéréosélective conduite en présence d'au moins un complexe organo-métallique tel que défini dans la revendication 16.

**20.** Procédé selon la revendication 19, dans lequel ladite réaction stéréosélective est choisie au sein du groupe consistant en réactions de réduction énantio- et/ou diastéréo-sélectives, hydroformylation, hydroboration, hydrosilylation, hydrocyanation, allylation, vinylation, et autres réactions de formation de la liaison C-C.

FIGURE 1

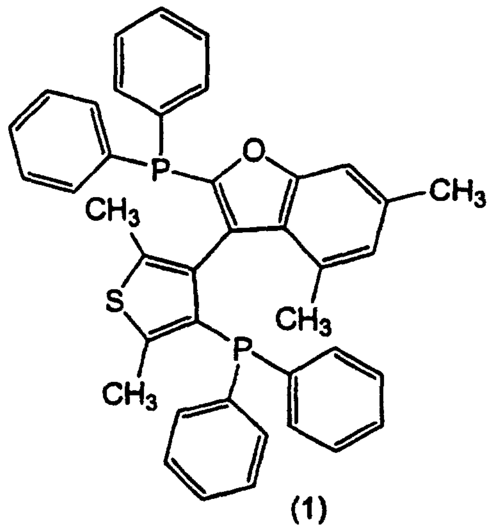

(1)

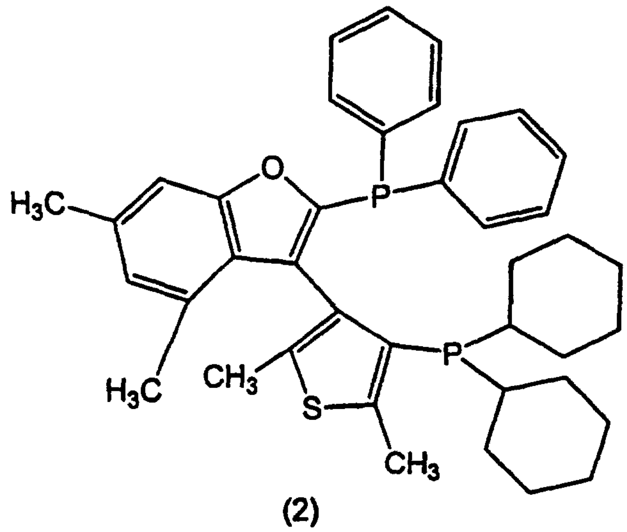

(2)

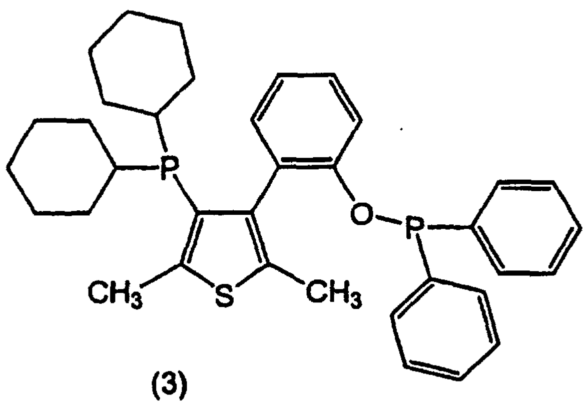

(3)

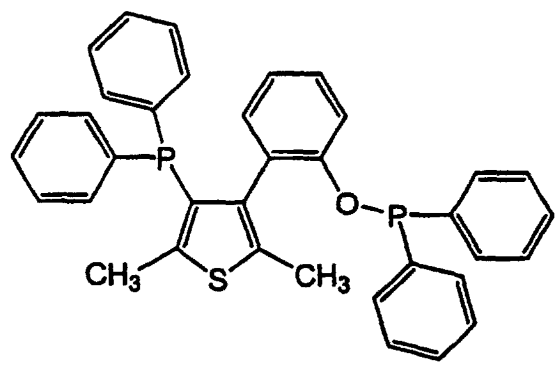

(4)

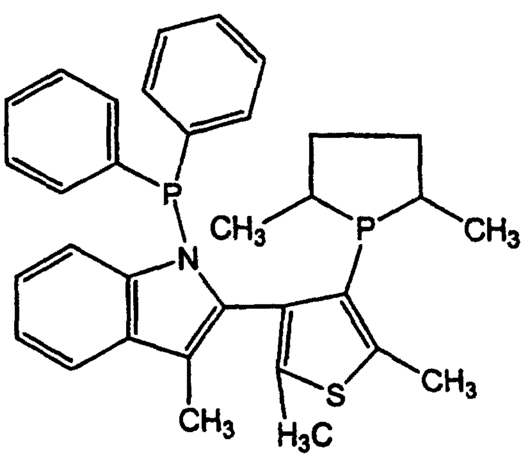

(5)

FIGURE 2

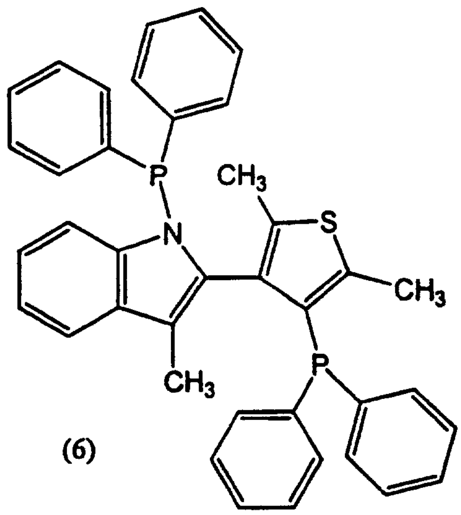

(6)

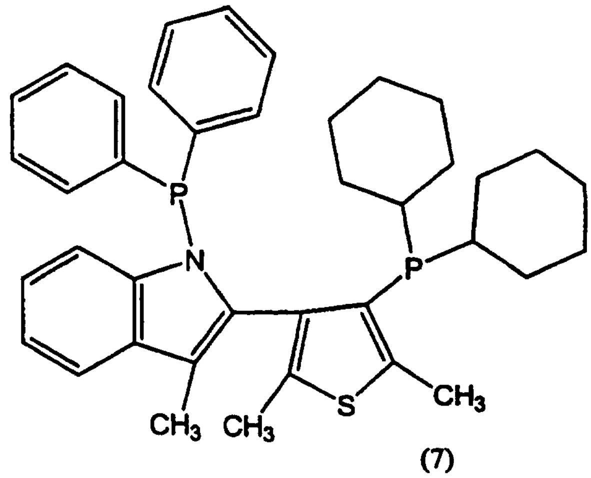

(7)

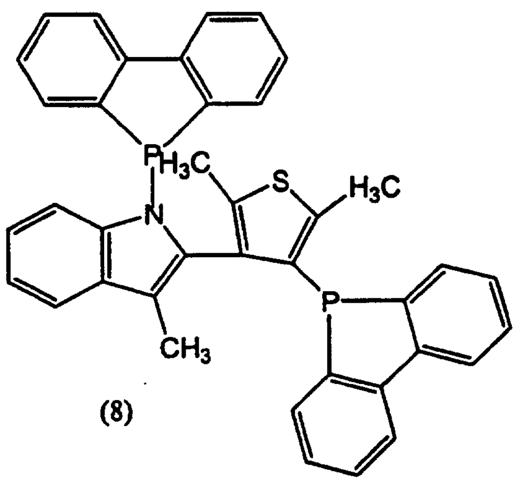

(8)

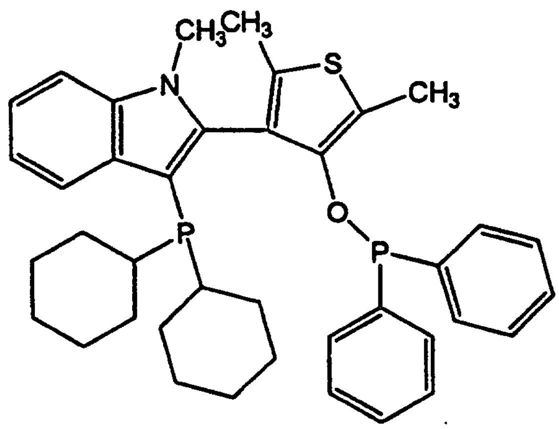

(9)

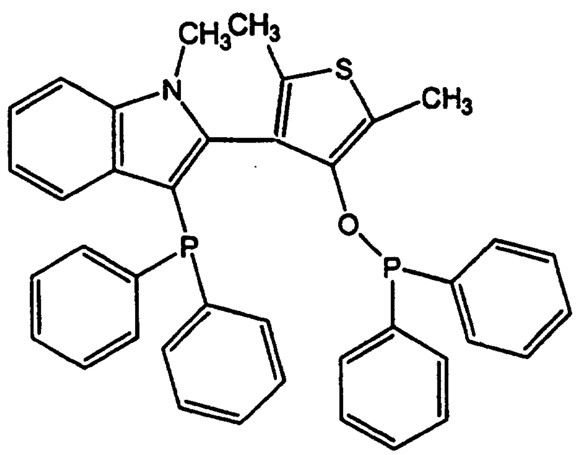

(10)

FIGURE 3

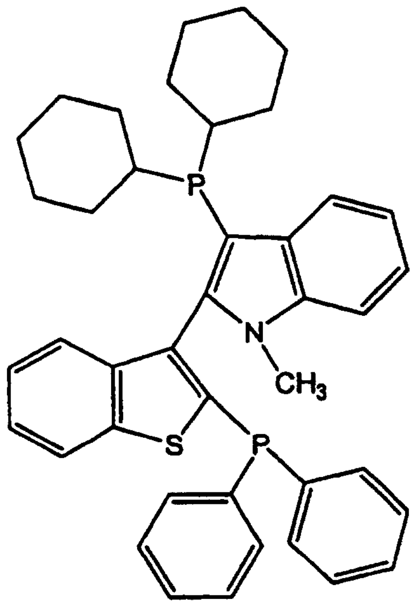

(11)

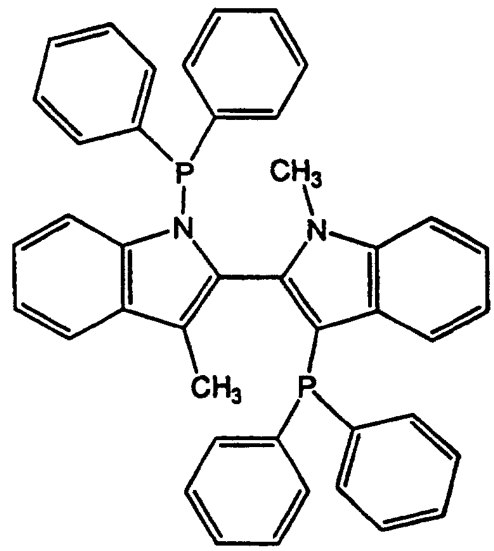

(12)

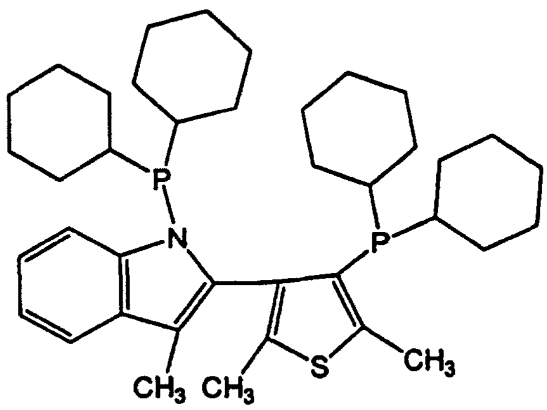

(13)

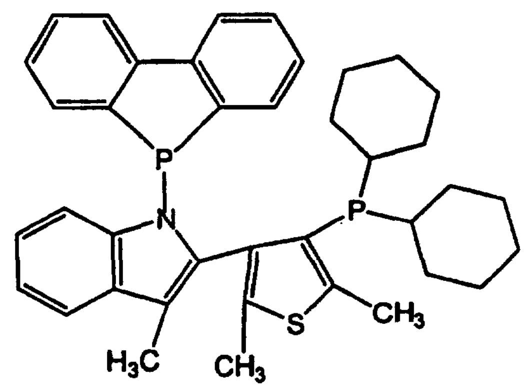

(14)

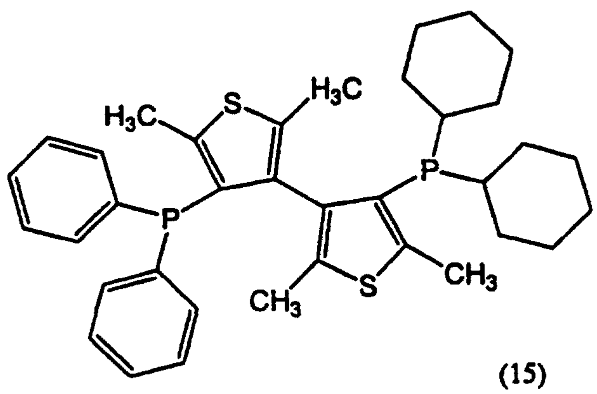

(15)